# EUROPEAN PATENT SPECIFICATION

(11) **EP 2 683 371 B1**
(45) Date of publication and mention of the grant of the patent: **21.10.2020**
(21) Application number: 12713772.7
(22) Date of filing: 09.03.2012
(51) Int. Cl.: A61K 31/167, A61K 31/18, A61K 31/19, A61K 31/343, A61K 31/4045, A61K 31/4184, A61K 31/501, A61P 3/10, A61P 3/00, A61P 7/02, A61P 9/10, A61P 13/12, A61P 37/00

(54) **COMPOUNDS AND METHODS FOR IMPROVING IMPAIRED ENDOGENOUS FIBRINOLYSIS USING HISTONE DEACETYLASE INHIBITORS**
VERBINDUNGEN UND VERFAHREN ZUR VERBESSERUNG VON GESTÖRTER ENDOGENER FIBRINOLYSE UNTER VERWENDUNG VON HISTONDEACETYLASE-HEMMERN
COMPOSÉS ET PROCÉDÉS D'AMÉLIORATION D'UNE FIBRINOLYSE ENDOGÈNE DYSFONCTIONNELLE À L'AIDE D'INHIBITEURS D'HISTONE DÉSACÉTYLASE

(30) Priority: 09.03.2011 US 201161464776 P; 09.03.2011 US 201161464809 P; 28.10.2011 US 201161628339 P
(43) Date of publication of application: 15.01.2014
(73) Proprietor: Cereno Scientific AB, 411 26 Gottenburg (SE)
(72) Inventor: LARSSON, Pia, 416 71 Göteborg (SE); BERGH, Niklas, 436 39 Askim (SE); JERN, Sverker, 459 33 Ljungskile (SE)
(74) Representative: Potter Clarkson
(86) International application number: PCT/GB2012/000229
(87) International publication number: WO 2012/120262

(56) References cited:
- EP-A1- 1 743 654
- WO-A1-03/013493
- WO-A2-02/055017
- WO-A2-2005/105066
- WO-A2-2007/030697
- WO-A2-2007/084775
- WO-A2-2007/115287
- WO-A2-2011/113013
- US-A1- 2006 178 437
- US-A1- 2009 270 497
- OJEMANN L M ET AL: "FIBRINOGEN AND VALPROIC-ACID", EPILEPSIA, vol. 22, no. 2, 1981, pages 242-243, XP009159014, & ANNUAL MEETING OF THE AMERICAN EPILEPSY SOCIETY, SAN DIEGO, CALIF., USA, NOV. 1980. EPILEPSIA. ISSN: 0013-9580
- SUTOR A H ET AL: "Influence of dipropylacetic acid (Ergenyl) on blood clotting", MEDIZINISCHE WELT 1974, vol. 25, no. 11, 1974, pages 447-449, XP009159016, ISSN: 0025-8512
- SCHLEEF R R ET AL: "Cytokine activation of vascular endothelium. Effects on tissue-type plasminogen activator and type 1 plasminogen activator inhibitor.", THE JOURNAL OF BIOLOGICAL CHEMISTRY 25 APR 1988, vol. 263, no. 12, 25 April 1988 (1988-04-25), pages 5797-5803, ISSN: 0021-9258

## Description

### FIELD OF INVENTION

The present invention generally relates to new medical uses, methods and compositions. More specifically it relates to improving or normalizing a suppressed endogenous vascular fibrinolysis, using different histone deacetylase inhibitors.

### BACKGROUND

Cardiovascular disease is the leading cause of morbidity and mortality in the western world and during the last decades it has also become a rapidly increasing problem in developing countries. An estimated 80 million American adults (one in three) have one or more expressions of cardiovascular disease (CVD) such as hypertension, coronary heart disease, heart failure, or stroke. Mortality data show that CVD was the underlying cause of death in 35% of all deaths in 2005 in the United States, with the majority related to myocardial infarction, stroke, or complications thereof. The vast majority of patients suffering acute cardiovascular events have prior exposure to at least one major risk factor such as cigarette smoking, abnormal blood lipid levels, hypertension, diabetes, abdominal obesity, and low-grade inflammation.

Pathophysiologically, the major events of myocardial infarction and ischemic stroke are caused by a sudden arrest of nutritive blood supply due to a blood clot formation within the lumen of the arterial blood vessel. In most cases, formation of the thrombus is precipitated by rupture of a vulnerable atherosclerotic plaque, which exposes chemical agents that activate platelets and the plasma coagulation system. The activated platelets form a platelet plug that is armed by coagulation-generated fibrin to form a blood clot that expands within the vessel lumen until it obstructs or blocks blood flow, which results in hypoxic tissue damage (so-called infarction). Thus, thrombotic cardiovascular events occur as a result of two distinct processes, i.e. a slowly progressing long-term vascular atherosclerosis of the vessel wall, on the one hand, and a sudden acute clot formation that rapidly causes flow arrest, on the other. This invention solely relates to the latter process.

Recently, inflammation has been recognized as an important risk factor for thrombotic events. Vascular inflammation is a characteristic feature of the atherosclerotic vessel wall, and inflammatory activity is a strong determinant of the susceptibility of the atherosclerotic plaque to rupture and initiate intravascular clotting. Also, autoimmune conditions with systemic inflammation, such as rheumatoid arthritis, systemic lupus erythematosus and different forms of vasculitides, markedly increase the risk of myocardial infarction and stroke.

Traditional approaches to prevent and treat cardiovascular events are either targeted 1) to slow down the progression of the underlying atherosclerotic process, 2) to prevent clot formation in case of a plaque rupture, or 3) to direct removal of an acute thrombotic flow obstruction. In brief, antiatherosclerotic treatment aims at modulating the impact of general risk factors and includes dietary recommendations, weight loss, physical exercise, smoking cessation, cholesterol- and blood pressure treatment etc. Prevention of clot formation mainly relies on the use of antiplatelet drugs that inhibit platelet activation and/or aggregation, but also in some cases includes thromboembolic prevention with oral anticoagulants such as warfarin. Post-hoc treatment of acute atherothrombotic events requires either direct pharmacological lysis of the clot by thrombolytic agents such as recombinant tissue-type plasminogen activator or percutaneous mechanical dilation of the obstructed vessel.

Despite the fact that multiple-target antiatherosclerotic therapy and clot prevention by antiplatelet agents have lowered the incidence of myocardial infarction and ischemic stroke, such events still remain a major population health problem. This shows that in patients with cardiovascular risk factors these prophylactic measures are insufficient to completely prevent the occurrence of atherothrombotic events.

Likewise, thrombotic conditions on the venous side of the circulation, as well as embolic complications thereof such as pulmonary embolism, still cause substantial morbidity and mortality. Venous thrombosis has a different clinical presentation and the relative importance of platelet activation versus plasma coagulation are somewhat different with an preponderance for the latter in venous thrombosis, However, despite these differences, the major underlying mechanisms that cause thrombotic vessel occlusions are similar to those operating on the arterial circulation. Although unrelated to atherosclerosis as such, the risk of venous thrombosis is related to general cardiovascular risk factors such as inflammation and metabolic aberrations.

Taken together, existing therapy and general risk factor management offers an insufficient protection against thrombotic events, both in the arterial and venous circulations, and cannot erase the severe consequences of such events. This prompts for development of novel preventive and therapeutic targets, especially more effective approaches that could prevent hazardous tissue ischemia even at such an early stage when symptoms have not yet occurred.

Interestingly, in an otherwise healthy individual, there is a natural "last line of defense" system, which can be activated if a clotting process, despite preventive measures, should occur in the vasculature. In brief, initiation of a thrombotic mechanism both on the arterial and venous sides of the circulation leads to activation of the innermost cell layer of the blood vessel (the endothelium), and as a response the cells rapidly release large amounts of the clot-dissolving substance tissue-type plasminogen activator (t-PA). This raises luminal t-PA levels to similar levels as with clinical thrombolytic therapy (i.e. administration of recombinant t-PA), but the potency of this endogenous response is 100-fold greater due to the extremely rapid onset of action.

Accumulating clinical, epidemiologic, and experimental data support the notion that if this thromboprotective function of the blood vessel wall is intact, it offers a powerful defense against formation of flow-arresting thrombi. Unfortunately, however, the capacity for acute t-PA release is impaired in several conditions with increased susceptibility to thrombotic events. These include atherosclerosis, hypertension, abdominal obesity, smoking, sedentary lifestyle, and low grade inflammation. This impairment is most likely due to a diminished synthesis and thereby reduced availability of the fibrinolytic activator in the endothelial cells.

In addition, we and others have shown that the efficiency of the endogenous fibrinolytic response is reduced in patients with increased risk for an atherothrombotic event, such as in atherosclerosis (Osterlund, B., et al. Acta Anaesthesiol Scand 52, 1375-1384 (2008), Newby, D.E., et al. Circulation 103, 1936-1941 (2001)). Recent data suggest that inflammation is a key underlying pathogenetic mechanism behind the suppressed t-PA production in this state. We have shown that prolonged exposure to the inflammatory cytokines tumor necrosis factor alpha (TNF-alpha) and interleukin-1 beta (IL-1b) causes a marked suppression of the transcription of t-PA (Ulfhammer, E., et al. Journal of Thrombosis and Haemostasis 4, 1781-1789 (2006), Larsson, P., et al. Thromb Res 123, 342-351 (2008)). Interestingly, it is known that the atherosclerotic plaque is associated with a local, potentially severe, inflammatory activation in the vessel wall and it is conceivable that this inflammatory milieu hampers the fibrinolytic response in the specific areas of the vasculature where it is pivotal to retain a high fibrinolytic capacity, thus increasing the risk of thrombotic events. Similarly, it is also likely that the increased incidence of thrombotic events in patients with systemic inflammatory conditions (e.g. autoimmune diseases and the metabolic syndrome), could also be related to a suppressive effect of circulating pro-inflammatory cytokines on t-PA synthesis.

Against this background, an alternative fourth approach to reduce the incidence of clinical thrombotic events should be to restore the capacity of the fibrinolytic "last line of defense system" in patients with an impairment of its function. Extensive efforts have been paid to find a feasible means for enhancing basal as well as stimulated endogenous fibrinolysis in subjects with a risk factor-associated reduction of fibrinolytic capacity. However, previous attempts to ameliorate t-PA synthesis with e.g. statins and retinoic acid have been disappointing. Other means of increasing fibrinolysis by blocking naturally occurring inhibitors of t-PA activity such as plasminogen activator inhibitor-1 (PAI-1) and carboxypeptidase U (CPU) have also been unsuccessful mainly due to limited drugability, such as poor pharmacokinetic properties of the drug candidates. Thus, so far no means have been described that could be used clinically to reverse an impairment of t-PA production.

We recently reported that the clinically used anti-seizure drug valproic acid (VPA) has a stimulatory effect on t-PA production at relatively high doses (Larsson, P., et al The epigenetic modifier valproic acid stimulates tissue-type plasminogen activator expression in human endothelial cells. Poster presented at Epigenetics 2009 (The epigenetics annual scientific conference 2009), Melbourne Australia (2009)). VPA is believed to inhibit histone deacetylase enzymes, i.e. be a so-called HDAC inhibitor (HDACi) that induces hyperacetylation of histones. This is an epigenetic control mechanism that changes chromatin structure, which makes DNA more accessible to the transcriptional machinery generally enhancing the transcription rate. We have now gathered experimental evidence indicating that t-PA production is largely controlled by this mechanism. Furthermore, VPA treatment of patients with epilepsy has recently been reported to lower the risk of atherothrombotic events by up to 40 % (Olesen, J.B., et al. Pharmacoepidemiol Drug Saf (2010)), an effect we believe is likely to be attributable to an increased fibrinolytic capacity in these patients after VPA treatment. Unfortunately, the plasma levels of VPA typically obtained during anticonvulsive VPA treatment (0.35-0.85mM) convey a risk of significant adverse side effects such as bleeding complications, pancreatitis, liver failure, weight gain etc. Hence, VPA in concentrations used in current clinical neurological or psychiatric practice precludes its use in primary and secondary prevention of cardiovascular disease because of its side effects. As stated by Olesen et al: "Although the risk/benefit ratio for the accepted epilepsy indications is favorable, the drug can have adverse effects and is clearly not suitable for cardiovascular prevention per se".

It has previously been shown that t-PA production in endothelial cells was increased when the cells were treated with the HDAC inhibitors Trichostatin A (TSA) and butyrate (Arts et al 1995, Biochem J. 1995 Aug 15;310 (Pt 1):171-6). However these substances are not suitable for clinical use due to toxicity and poor pharmacokinetic properties, and hence potential *in vivo* use was never discussed. Recently, this work was extended to describe the cell signaling mechanisms behind the up-regulation of t-PA after TSA, butyrate and MS-275 treatment in cultured endothelial cells (Dunoyer-Geindre and Kruithof, Cardiovascular Research 90(3) 457-63 (2011)). In this reference the authors make the following comment regarding the potential side effect on t-PA when epigenetic modifiers are used in cancer therapy: "it is likely that therapeutic use of inhibitors of DNA methylation or of HDAC inhibitors has an impact on expression of t-PA *in vivo".* However, there was no suggestion that such substances could be used as a preventive therapy to specifically target an impaired t-PA production in order to reduce the risk of cardiovascular events. Moreover, the substances investigated in the latter study are either precluded from clinical use due to toxicity (TSA) or have only been shown to be effective in doses that are too high to be used in cardiovascular prevention (butyrate and MS-275). On a general note, to our knowledge no data has previously been presented to show that HDAC inhibitors can significantly augment t-PA production at concentrations low enough to permit clinical usage as prophylactic agents against cardiovascular events without significant or intolerable side effects.

Recently, we investigated the effect of low concentrations of VPA on t-PA production when suppressed by pro-inflammatory stimuli. We surprisingly found that VPA is an effective t-PA inducing agent already at sub-clinical concentrations and that low concentrations surprisingly are enough to markedly increase or normalize an inflammation-suppressed t-PA production. We therefore believe that VPA indeed is useful for cardiovascular disease prevention at these low concentrations in patients with inflammation-suppressed t-PA production. The side effects found using higher concentrations/doses of VPA previously known in the art in e.g. antiepileptic treatment makes, as has been previously mentioned, VPA unsuitable for primary and secondary prevention of cardiovascular disease. We have solved this problem by using the unexpectedly low concentrations/doses of VPA, described in this application, to increase or normalize an inflammation-suppressed t-PA production.

Since TNF-alpha is a very potent cell activator with profound effects on multiple cellular functions, including both transcriptional and posttranscriptional regulatory mechanisms as well as signaling pathways, it was impossible to predict if VPA at all could have any effect on t-PA expression in TNF-suppressed cells (this consideration also applies to the new generation of HDACi, as described herein). However, we surprisingly found that unexpectedly low concentrations VPA could completely off-set the inhibition of TNF-alpha on the expression of t-PA. Interestingly, the concentrations needed to reverse the effect of TNF-alpha were in a range more suitable for cardiovascular prevention (below 0.35 mM). This strong capability of VPA to restore t-PA production in TNF-treated endothelium makes it possible to use low doses of VPA for an efficient prophylactic treatment with relatively few side effects to improve the endogenous fibrinolysis in patients with local or systemic inflammation. It has not previously been shown that VPA can counteract this inflammation-suppression of t-PA. Furthermore, when this effect is seen at surprisingly low concentrations our invention makes it possible to use this treatment for preventing cardiovascular disease without intolerable side effects.

We even more surprisingly found that, at higher concentrations TNF-alpha actually potentiated the stimulatory effect of VPA on the production of t-PA. Hence, exposure of endothelial cells to TNF-alpha caused a profound change of the pattern of the VPA dose-response curve, with a markedly augmented maximum efficacy response to VPA. This unexpected finding indicates that there is a complex interaction between the cellular effects of the two agents, which may also explain the fact that much lower concentrations than anticipated were sufficient to increase or normalize an inflammation-suppressed fibrinolytic function. Again, this supports the notion that it is possible to use VPA for preventive treatment against cardiovascular disease in these patients without the adverse side effects seen in e.g. antiepileptic treatment.

The amplified cellular t-PA production in response to VPA further supports the notion that even in atherosclerosis, where a highly inflamed microenvironment is present around the plaque, low doses of VPA are sufficient to restore an inflammation-suppressed fibrinolytic function. These new observations indicate that low or sub-clinical doses of VPA are sufficient to restore an impaired t-PA production that is suppressed by inflammatory stress.

In U.S. patent application number US 2009/0270497, methods are described for treating systemic non-localized inflammatory conditions, mainly sepsis, by administering a therapeutically effective amount of a compound that is a pan-HDAC inhibitor. Many substances are described in this application, including VPA. However, the application is related to the specific treatment of the inflammatory condition as such, and a potential stimulation of the endogenous thromboprotective response is not mentioned. Furthermore, the ability of VPA at low concentrations to normalize an inflammation-suppressed t-PA production is not mentioned in the application.

Recently, a number of more specific HDAC inhibitors have been developed, which by virtue of their greater specificity are more potent and efficient in lower doses. For instance, whereas VPA is efficient in the mM range, the new-generation HDAC inhibitors usually cause similar HDAC inhibition in the low µM range. Furthermore, the newer substances are developed to optimize pharmacokinetics as well as to reduce toxicity. However, new-generation HDACis in doses used for cancer treatment are still associated with adverse side effects that preclude their use in cardiovascular preventive treatment.

However our new observations unexpectedly, but clearly, display that use of substantially lower concentrations of the HDACi than those used in clinical cancer therapy cause a significant increase of t-PA production. Since surprisingly low concentrations of these HDACi substances are enough to increase or normalize an impaired t-PA production (due to e.g. inflammation or genetic factors), treatment at these concentrations is likely to be associated with markedly fewer and less severe adverse side-effects than those found in clinical cancer therapy. Therefore, these HDACi substances have now been found to be suitable for prophylactic treatment against thrombotic cardiovascular disease at these low concentrations. In this way, we have solved the problem of adverse side effects, thus making it possible to use these substances for cardiovascular preventive treatment.

We also surprisingly found that low concentrations of HDACi could completely off-set the inhibition of TNF-alpha on the expression of t-PA. Indeed, the concentrations needed were in a range believed to be suitable for cardiovascular prevention (e.g. for Belinostat approximately 0.05-0.2 µM). Of note, the effect on t-PA expression was not explained by an antiinflammatory action per se, but was clearly mediated by effects on non-inflammatory pathways (see Example 78). The strong capability of the HDACis to restore t-PA production in TNF-alpha treated endothelium makes it possible to use low doses for an efficient prophylactic treatment with relatively few side effects in order to improve the endogenous fibrinolysis in patients with local or systemic inflammation.

To our knowledge, it has not previously been shown that HDACi substances can counteract this inflammation-suppression of t-PA. Furthermore, when this effect is seen at very low concentrations our invention makes it possible to use this treatment for preventing cardiovascular disease without intolerable side effects in patients with impaired endogenous fibrinolysis due to local or systemic inflammation. These new observations indicate that low doses of HDACi are sufficient to restore an impaired t-PA production.

The different HDACis described in this application belong to different structural classes (e.g. hydroxamates, benzamides, and cyclic peptides) and could have selectivity for different HDAC isoforms. The hydroxamates (e.g. Vorinostat, Belinostat, Givinostat, Panobinostat, PCI-24781, JNJ26481585, and SB939) are pan-HDACis, i.e. they inhibit HDACs of different isoforms with relatively similar efficiency, although differences in HDAC enzyme selectivity exist within the different structural classes. The benzamides (including Mocetinostat and CXD101) are probably more selective for inhibition of the HDAC Class I and II isoforms (Class I: HDAC1, 2, 3 and 8 and Class II: HDAC4, 6, 7 and 9). The differences among the different HDACi lead to unpredictable differences in their regulation of endothelial cell gene expression. For example, the regulation of E-selectin is hard to predict since Mocetinostat strongly induces expression, Givinostat strongly suppresses expression, while VPA and Belinostat have almost no effect on the regulation of the gene.

However, to our surprise we found that the HDACi substances described in this application had similar qualitative inducing effects on t-PA production. Furthermore, this effect is seen at unexpectedly low concentrations for all HDACi substances, even though they belong to different chemical classes and have different selectivity profiles. Hence, these data indicate that t-PA is sensitive to HDAC inhibition as such, not the individual molecules. Interestingly, however, we found that substances of the new-generation hydroxamate class were even more potent t-PA inducers at very low concentrations, as demonstrated in Example 77, making this class even more preferred as stimulators of endogenous t-PA production.

These novel approaches are the first clinically feasible strategies to normalize a defective vascular fibrinolysis in patients prone to atherothrombotic events due to reduced t-PA production. Hence, treatment with low doses of HDACi improves the "last line of defense" against thrombotic events such as myocardial infarction, ischemic stroke or venous thrombosis when such events are triggered despite optimal traditional risk factor therapy.

WO 2007/084775 describes methods of treating autoimmune disorders, coronary artery disease, allergy symptoms, allograft rejection sepsis/toxic shock.

Ojemann, L. M., et al., Epilepsia, 22(2), 242-243, (1980) is entitled "Fibrogen and Valpoic-Acid".

Sutor, A. H., et al., Medizinische Welt, 25(11), 447-449, (1974) is entitled "Influence of dipropylacetic acid (Ergenyl) on blood clotting".

### SUMMARY OF THE INVENTION

Certain HDACi substances have been found to be surprisingly efficient at low concentrations to restore a suppressed fibrinolytic function, making these substances suitable for prophylactic or acute treatment to reduce the risk of clinical arterial or venous thrombotic events. Furthermore, it has not previously been shown that HDACi substances can counteract inflammation-suppressed t-PA production. When the effect on t-PA production is seen at surprisingly low concentrations our invention makes it possible to use this treatment for preventing cardiovascular disease without the adverse side effects observed in other diseases, e.g. cancer, at higher concentrations. This is very important since it solves the problem that there are higher demands when it comes to few and tolerable side effects for prophylactic treatment of large patient groups as is the case for cardiovascular disease prevention in patients with e.g. inflammation-suppressed fibrinolytic function using the HDACi substances described in the application.

Described herein is a way to use these HDACi substances at low concentrations to improve a suppressed endogenous fibrinolysis.

Also described herein is a way to use these HDACi substances in low concentrations as a safe and effective prophylactic and/or acute treatment with few side effects to improve a suppressed endogenous fibrinolysis and hence reduce thrombosis in humans.

Also described herein is a way to use these HDACi substances at low concentrations to restore an inflammation-suppressed fibrinolytic function.

Also described herein as a way to use these HDACi substances in low concentrations as a safe and effective prophylactic and/or acute treatment with few side effects to improve an endogenous fibrinolysis impaired by local or systemic inflammation in humans.

Also described herein is a way is to use these HDACi substances in low concentrations as a safe and effective prophylactic and/or acute treatment with few side effects to improve endogenous fibrinolysis in patients diagnosed with atherosclerosis.

Also described herein as a way to use these HDACi substances in low concentrations as a safe and effective prophylactic and/or acute treatment with few side effects to improve endogenous fibrinolysis in patients with a diagnosed local or systemic inflammation.

Also described herein as a way to use these HDACi substances in low concentrations as a safe and effective prophylactic and/or acute treatment with few side effects to improve endogenous fibrinolysis in patients with a biomarker profile (one or several biomarkers) indicative of local or systemic inflammation.

Also described herein as a way to use these HDACi substances in low concentrations as a safe and effective prophylactic and/or acute treatment with few side effects to improve endogenous fibrinolysis in patients displaying elevated TNF-alpha levels.

Valproic acid has been found to be surprisingly efficient at low concentrations to restore an inflammation-suppressed fibrinolytic function, making it possible to use low concentrations of valproic acid to reduce the risk of thrombotic cardiovascular events in patients with inflammation-suppressed fibrinolytic function. It has not previously been shown that VPA can counteract inflammation-suppressed t-PA production. Furthermore, when this effect is seen at surprisingly low concentrations our invention makes it possible to use this treatment for preventing cardiovascular disease without the adverse side effects observed in other diseases treated with VPA at higher concentrations. This is very important since it solves the problem of higher demands regarding side effects for prophylactic treatments, where the side effects must be few and tolerable. Thus, making prophylactic treatment of large patient groups possible, as is the case for cardiovascular disease prevention in patients with inflammation-suppressed fibrinolytic function using VPA. The finding that the maximum efficacy of VPA on t-PA production was markedly augmented when endothelial cells were exposed to TNF-alpha further displays that there is an unexpected non-linear relationship between VPA, TNF-alpha and t-PA. We believe that this relationship means that we can use even lower doses than we first anticipated, based on our initial results on TNF-suppressed t-PA production, to increase or normalize an inflammation-suppressed fibrinolytic function. This further improves the side effect profile and makes VPA even more suitable for preventive treatment against cardiovascular disease in patients with inflammation-suppressed fibrinolytic function.

A primary object of the present invention is to use valproic acid in low concentrations to improve or normalize endogenous fibrinolysis impaired by local or systemic inflammation.

Another object of the present invention is to use valproic acid in low concentrations as a safe and effective prophylactic and/or acute treatment with few side effects to improve or normalize endogenous fibrinolysis impaired by local or systemic inflammation in humans.

Another object of the present invention is to use valproic acid in low concentrations as a safe and effective prophylactic and/or acute treatment with few side effects to improve or normalize endogenous fibrinolysis in patients with a diagnosed local or systemic inflammation.

Another object of the present invention is to use valproic acid in low concentrations as a safe and effective prophylactic and/or acute treatment with few side effects to improve or normalize endogenous fibrinolysis in patients with a biomarker profile (one or several biomarkers) indicative of local or systemic inflammation.

Another object of the present invention is to use valproic acid in low concentrations as a safe and effective prophylactic and/or acute treatment with few side effects to improve or normalize endogenous fibrinolysis in patients displaying elevated TNF-alpha levels.

Another object of the present invention is to use valproic acid in low concentrations as a safe and effective prophylactic and/or acute treatment with few side effects to improve or normalize endogenous fibrinolysis in patients diagnosed with atherosclerosis.

Other objects and advantages of the present invention will become obvious to the reader and it is intended that these objects and advantages are within the scope of the present invention.

### BRIEF DESCRIPTION OF DRAWINGS

**Figures 1A and 1B** shows dose-response curves for Belinostat and Vorinostat, respectively, on t-PA mRNA expression in human endothelial cells. One representative experiment is shown.
**Figure 2** is a graph that shows the ability of Belinostat and Vorinostat to counter-act a TNF-alpha mediated suppression of t-PA at low concentrations in human endothelial cells. One representative experiment is shown.

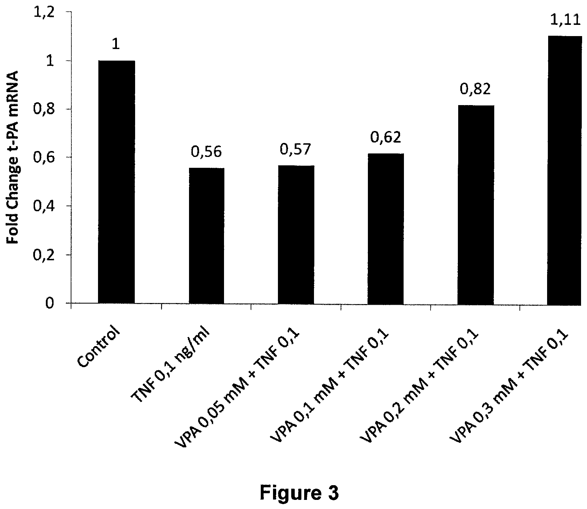

**Figure 3** shows the ability of low concentrations of VPA to counteract TNF-alpha mediated t-PA suppression in HUVEC cells. One representative experiment is shown.

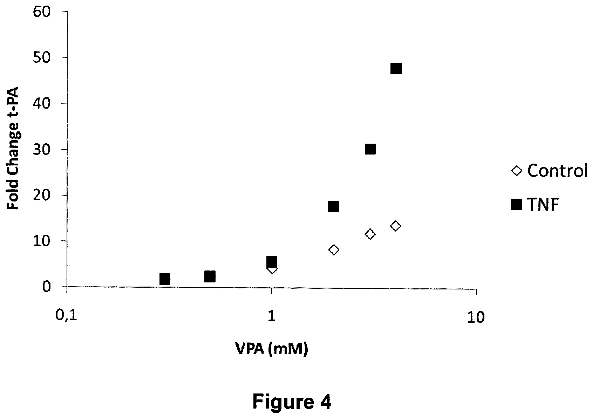

**Figure 4** is a graph that shows the dose-response curves for VPA (0.3-4 mM) in the presence or absence of TNF-alpha (10 ng/ml). One representative experiment is shown.

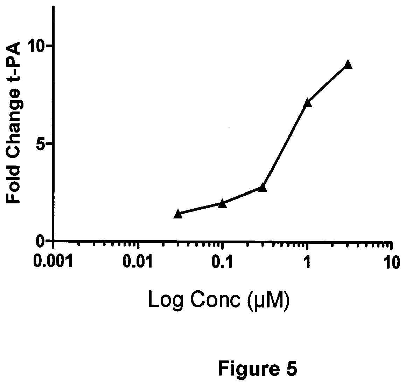

**Figure 5** shows a dose-response curve for Vorinostat on t-PA mRNA expression in HUVEC after 24 h incubation (n=3)

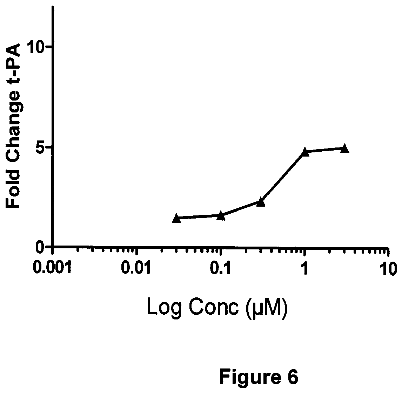

**Figure 6** shows a dose-response curve for Belinostat on t-PA mRNA expression in HUVEC after 24 h incubation (n=3)
**Figure 7** shows a dose-response curve for Givinostat on t-PA mRNA expression in HUVEC after 24 h incubation (n=3)
**Figure 8** shows a dose-response curve for JNJ-26481585 on t-PA mRNA expression in HUVEC after 24 h incubation (n=3)
**Figure 9** shows a dose-response curve for SB939 on t-PA mRNA expression in HUVEC after 24 h incubation (n=3)
**Figure 10** shows a dose-response curve for Panobinostat on t-PA mRNA expression in HUVEC after 24 h incubation (n=3)

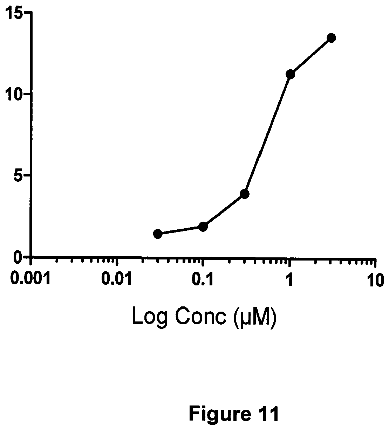

**Figure 11** shows a dose-response curve for Mocetinostat on t-PA mRNA expression in HUVEC after 24 h incubation (n=3)
**Figure 12** shows a dose-response curve for PCI-24781 on t-PA mRNA expression in HUVEC after 24 h incubation (one representative experiment)
**Figure 13** shows the effect of TNF-alpha (TNF-a), givinostat and the prototypical anti-inflammatory substances acetylsalicylic acid (ASA) and ibuprofen (IBU) on t-PA expression (one representative experiment).

### DETAILED DESCRIPTION OF INVENTION

The present invention relates to fibrin degradation or breakdown (also called fibrinolysis), and compositions and methods for the treatment of pathological conditions associated with excess fibrin deposition and/or thrombus formation. In particular, the present invention relates to fibrin degradation or breakdown, and compositions and methods for the treatment of pathological conditions associated with excess fibrin deposition and/or thrombus formation, particularly when due to an impaired fibrinolysis. More particularly, the present invention relates to fibrin degradation or breakdown, and compositions and methods for the treatment of pathological conditions associated with excess fibrin deposition and/or thrombus formation, when due to an impaired fibrinolysis caused by reduced endogenous t-PA production. Also described herein is a new method for potentiating the degradation of fibrin deposits and preventing such deposits associated with pathological conditions or which may lead to such conditions.

There is described herein administering to a subject in need of such treatment a therapeutically effective amount of an HDAC inhibitor, such as any of the HDAC inhibitors described in the application, such as Vorinostat (SAHA), Belinostat (PXD-101), Givinostat (ITF2357), Panobinostat (LBH 589), PCI-24781, JNJ-26481585, SB939, Mocetinostat (MGCD0103), or CXD 101, which compounds can be used alone or in combination (e.g. in combination with each other), or in combination with the HDAC inhibitor Valproic acid (VPA), and optionally in association with one or more pharmaceutically acceptable carriers or excipients and/or one or more drugs targeting clot formation.

The present invention provides valproic acid, or a pharmaceutically acceptable salt thereof, for use in:
(I) improving or normalizing endogenous fibrinolysis impaired by local or systemic inflammation; or
(II) treating or preventing a pathological condition associated with excess fibrin deposition and/or thrombus formation, wherein the pathological condition is caused wholly or at least in part by an increased fibrin deposition and/or reduced fibrinolytic capacity due to local or systemic inflammation,
wherein the valproic acid, or a pharmaceutically acceptable salt thereof, is administered in an amount between 1 µg to 30 mg per kilogram of body weight per day.

In the present application, the terms 'fibrinolysis' and 'fibrinolytic system' are used not only to refer to specific components and actions of the fibrinolytic system as such, but can optionally include other physiological functions and agents that interact with the fibrinolytic system, such as platelets and products released from them and components of the plasma coagulation system.

Pathological conditions, which may be treated in accordance with the invention are those which are caused wholly or at least in part by an increased fibrin deposition and/or reduced fibrinolytic capacity. These include but are not limited to atherosclerosis, myocardial infarction, ischemic stroke, deep vein thrombosis, pulmonary embolism, disseminated intravascular coagulation, renal vascular disease, and intermittent claudication. Also, in another disclosure the substances are used in conditions that, through their suppressive effect on the vascular fibrinolytic system, increase the risk for the above-mentioned disease states. Such conditions include but are not limited to hypertension, obesity, diabetes, the metabolic syndrome, and cigarette smoking. In addition, our invention can be used in subjects with a fibrinolytic activity that is reduced for other reasons, including but not limited to inherited variations in components of the fibrinolytic system.

As discussed above, thrombotic cardiovascular events occur as a result of two distinct processes, i.e. a slowly progressing long-term vascular atherosclerosis of the vessel wall, on the one hand, and a sudden acute clot formation that rapidly causes flow arrest, on the other. Particular pathological conditions that may be treated are those relating to the latter process.

In particular, the pathological condition treated may be selected form the group consisting of myocardial infarction, stable angina pectoris, unstable angina pectoris, intermittent claudication, ischemic stroke, transient ischemic attack, deep vein thrombosis, and pulmonary embolism.

More particularly, the pathological condition is selected from the group consisting of deep vein thrombosis and pulmonary embolism.

In addition, pathological conditions that can be treated in accordance with the invention are those that are caused wholly or at least in part by an increased fibrin deposition and/or reduced fibrinolytic capacity due to local or systemic inflammation. These include but are not limited to atherosclerosis, the metabolic syndrome, diabetes, disseminated intravascular coagulation, rheumatoid arthritis, glomerulo-nephritis, systematic lupus erythematosis, vasculitides, autoimmune neuropathies, and granulomatous disease as well as inflammation associated with other conditions (such as the metabolic syndrome, diabetes, disseminated intravascular coagulation, rheumatoid arthritis, glomerulo-nephritis, systematic lupus erythematosis, vasculitides, autoimmune neuropathies, and granulomatous disease as well as inflammation associated with other conditions).

In a further preferred embodiment pathological conditions that can be treated in accordance with the invention are those that are caused wholly or at least in part by an increased fibrin deposition and/or reduced fibrinolytic capacity due to local or systemic inflammation. These include but are not limited to myocardial infarction, stable angina pectoris, unstable angina pectoris, intermittent claudication, ischemic stroke, transient ischemic attack, deep vein thrombosis, and pulmonary embolism.

In a particularly preferred aspect of the invention, the pathological condition is selected from the group consisting of deep vein thrombosis and pulmonary embolism.

In addition to traditional diagnosis of a systemic or local inflammation by a physician as is known in the art, a local or systemic inflammation can be determined in patients using one or more biomarkers coupled to inflammation. These biomarkers include, but are not limited to, C reactive protein, TNF-alpha, high sensitive C-reactive protein (hs-CRP), fibrinogen, IL-1 beta, and IL-6. Particular methods for determining whether a patient has systemic or local inflammation include those described hereinafter.

In addition, atherosclerotic plaques are known to be associated with a very localized inflammatory process. Hence, local inflammation may also be indirectly determined by the presence of atherosclerotic plaques as diagnosed by vascular ultrasound or other imaging techniques.

It will be understood that whether a patient is suffering from impaired fibrinolysis and/or reduced endogenous t-PA production may be easily determined by the skilled person.

The skilled person will understand that, to identify a poor level of fibrinolysis in a patient (i.e. reduced fibrinolytic capacity), there are a few different alternatives available. For example, high circulating levels of PAI-1 (the main inhibitor of t-PA) are generally considered to be indicative of poor fibrinolysis, and this can be measured by commercially available methods (Coaliza® PAI-1 (Chromgenix), TriniLIZE® PAI-1 (Trinity Biotech), Imubind® Plasma PAI-1 (American Diagnostica), Zymutest PAI-1 (Hyphen Biomed)). Further, low systemic levels of free, active t-PA is also an indicator of general poor fibrinolysis and can also be measured by commercial methods (TriniLIZE® t-PA antigen and activity (Trinity Biotech), as is the presence of a low-producer (T) genotype of the t-PA -7351 C/T polymorphism. Functional assays measuring clot lysis time have also been used to assess global fibrinolysis (Thrombinoscope™ (Synapse, BV, Maastricht, the Netherlands), IL/ROTEM® (Term International GmbH , Munich, Germany), TEG® (Haemoscope, Niles), CloFAL assay (Peikang Biotechnology Co. Ltd. Shanghai, China)).

In addition, local production and release of t-PA can be determined by regional models. Normally, this is performed in a model vascular bed, e.g. the human forearm *(*Hrafnkelsdottir T, et al (2004) Regulation of local availability of active tissue-type plasminogen activator in vivo in man. J Thromb Haemost 2: 1960-1968*)* where a catheter is placed in the brachial artery and a vein and the amount of t-PA released over the forearm vascular bed after agonist induced release is measured.

In particular disclosures, the pathological condition is selected from the group consisting of atherosclerosis, myocardial infarction, ischemic stroke, deep vein thrombosis, pulmonary embolism, disseminated intravascular coagulation, renal vascular disease, and intermittent claudication.

More particularly, the pathological condition is selected form the group consisting of myocardial infarction, stable angina pectoris, unstable angina pectoris, intermittent claudication, ischemic stroke, transient ischemic attack, deep vein thrombosis, and pulmonary embolism.

More particularly, the pathological condition is selected from the group consisting of deep vein thrombosis and pulmonary embolism.

More particularly, the pathological condition is selected from conditions that, through their suppressive effect on the vascular fibrinolytic system, increase the risk for the above-mentioned disease states. Such conditions include but are not limited to hypertension, obesity, diabetes, the metabolic syndrome, and cigarette smoking.

In particular, the patient has a fibrinolytic activity that is reduced for reasons other than those provided in respect of the embodiment of the invention mentioned directly above (e.g. other than hypertension, obesity, diabetes, the metabolic syndrome, and cigarette smoking), including but not limited to inherited variations in components of the fibrinolytic system.

As discussed above, it has also been found that pathological conditions that can be treated in accordance with the invention are those that are caused wholly or at least in part by an increased fibrin deposition and/or reduced fibrinolytic capacity due to local or systemic inflammation. In particular, we have shown that the prototypical anti-inflammatory substances aspirin (ASA) and ibuprofen (IBU) are unable to reverse the suppression of t-PA caused by inflammatory stress (see example 78). Therefore the effect of HDACi on inflammatory suppression of t-PA is unlikely to be a result of a general anti-inflammatory effect of these substances.

The skilled person will understand that whether the patient the increased fibrin deposition and/or reduced fibrinolytic capacity is due to "local or systemic inflammation" as used herein can be determined using one or more biomarkers coupled to inflammation, including but not limited to C reactive protein, TNF-alpha, high sensitive C-reactive protein (hs-CRP), fibrinogen, IL-1beta, and IL-6 (e.g. by increased concentration of one or more of these biomarkers in relation to control levels as known in the art). Commercial analytical platforms that can be used to quantify these biomarkers include, but are not limited to, Afinion™ (Medinor AB, Sweden), CA-7000 (Siemens Healthcare Diagnostics Inc, NY, US), Immulite® 2000 Immunoassay System (Siemens Healthcare Diagnostics Inc).

Particular biomarkers that may identify local or systemic inflammation include high sensitive C-reactive protein (hs-CRP) (at or above 2.0 mg/l serum) and fibrinogen (at or above 3g/l serum) (Corrado E., et al. An update on the role of markers of inflammation in atherosclerosis, Journal of atherosclerosis and Thrombosis, 2010;17:1-11, Koenig W., Fibrin(ogen) in cardiovascular disease: an update, Thrombosis Haemostasis 2003;89:601-9).

Whether the patient has a local or systemic inflammation that can be determined using one or more biomarkers coupled to inflammation, including but are not limited to C reactive protein, TNF-alpha, high sensitive C-reactive protein (hs-CRP), fibrinogen, IL-1beta, and IL-6 (e.g. by increased concentration of one or more of these biomarkers in relation to control levels as known in the art).

Whether the patient has a local or systemic inflammation that can be determined by identifying the presence of high sensitive C-reactive protein (hs-CRP) (at or above 2.0 mg/l serum) and/or fibrinogen (at or above 3g/l serum).

In particular, the patient has local inflammation that may be indirectly determined by the presence of atherosclerotic plaques as diagnosed by vascular ultrasound or other imaging techniques.

In certain disclosure herein, the compound is valproic acid, or a pharmaceutically-acceptable salt thereof.

As described herein, there is provided valproic acid, or a pharmaceutically acceptable salt thereof, for use in treating or preventing a pathological condition associated with excess fibrin deposition and/or thrombus formation, wherein the pathological condition is caused wholly or at least in part by an increased fibrin deposition and/or reduced fibrinolytic capacity due to local or systemic inflammation.

In particular disclosures, the dose of valproic acid, or pharmaceutically acceptable salt thereof, is as described below.

In a further disclosure related to the disclosure mentioned directly above, the pathological condition is cardiovascular disease.

As used herein, "therapeutically effective amount" means an amount of an agent which confers the required pharmacological or therapeutic effect on a subject without undue adverse side effects. It is understood that the therapeutically effective amount will vary from subject to subject. The amounts of and dosage regimes of the HDACi covered in this application, which are administered to a subject to normalize or increase fibrinolysis, will depend on a number of factors such as the substance of choice, mode of administration, the nature of the condition being treated, age, body weight and general condition of the subject being treated, and the judgment of the prescribing physician. The HDACi substances covered in this application can be given as a specific dose at a specific interval based on these factors. Alternatively, as there can be a significant inter-individual variation in the plasma concentrations reached with a specific dose of these substances, the concentration in plasma can be continuously monitored and the patient titrated to reach a specific dose and interval that results in a desired plasma concentration. Examples of dosing intervals for the HDACi substances in this application include, but are not limited to, administration once daily or administration divided into multiple daily doses. The administration may be continuous, i.e. every day, or intermittent. The term intermittent, as used herein, means stopping and starting at either regular or irregular intervals. For example, intermittent administration of an HDACi may be administration one to six days per week, or it may mean daily administration for two weeks followed by one week without administration, or it may mean administration on alternate days. Generally speaking, the HDACi may be administered in an amount where the fibrinolysis is increased or normalized without undue adverse side effects making it suitable for both prophylactic and acute treatment.

Surprisingly, we have found that the dose required is significantly lower than the standard dose used in e.g. oncology applications. By achieving an increase or normalization of the t-PA production already at these low doses we solve the problem of side effects that precludes the use of these substances at higher doses for cardiovascular prevention treatment.

Generally, the dose described herein (e.g. for thrombosis prevention) is <50% (e.g. 0.1 to 49.9%, such as 1 to 40%, 2 to 30%, 5 to 25% or even 1 to 25%) by weight (w/w) of that used for oncology indications. More preferably, the dose used is <20% by weight of that used for oncology indications. Most preferably, the dose is ≤10% by weight of that used for oncology indications. Similar, limitations apply to the dose as a percentage of the maximum tolerated dose (MTD).

Also described herein, the compound is administered in a dose that is <50% (e.g. 0.1 to 49.9%, such as 1 to 40%, 2 to 30%, 5 to 25% or even 1 to 25%) by weight of:
(i) that used for oncology indications; or
(ii) the maximum tolerated dose.

In particular, the dose is <20% by weight (e.g. 0.1 to 19.0%, such as 5 to 15% or even 1 to 15%) or, more preferably, ≤10% by weight (e.g. 0.1 to 10.0%, such as 1 to 5% or even 1 to 10%) of that used for oncology indications or of the maximum tolerated dose.

For the avoidance of doubt, the reference to the dose that is "used" in respect of oncology applications or to the maximum tolerated dose includes doses that are indicated as such in the relevant literature (i.e. the literature associated with the oncology application of that compound and/or literature associated with clinical trials conducted in respect of such compounds). In this regard, particularly preferred compounds of the invention are those that have been the subject of clinical trials (e.g. for use in oncology).

Generally speaking, the HDACi substances described in this application may be administered in an amount of 0.01-1000 mg/day, typically yielding a maximum plasma concentration (Cmax) of 0.1 nM to 10 µM. Preferably, the amount administered should be in the range of 0.1-1000 mg/day, typically a Cmax of 1 nM to 10 µM. More preferably, the amount administered should be between 0.1-300 mg/day, typically yielding a Cmax of 1 nM to 1 µM. Most preferably, the amount administered should be between 0.1-100 mg/day, typically yielding a Cmax of 1 nM to 0.5 µM.

The plasma concentrations described in this application can be achieved by a dose titration for each substance as is known in the art. Examples of this type of titration are described in Examples 66-69.

Also described herein, there is provided a method, compound for use or use as defined in respect of any one or more of the preceding aspects of the invention, wherein the compound is administered in an amount of 0.01-1000 mg/day, preferably yielding a Cmax of 0.1 nM to 10 µM.

In particular, the amount administered should be in the range of 0.1-1000 mg/day, preferably yielding a Cmax of 1 nM to 10 µM.

More particularly, the amount administered should be in the range of 0.1-300 mg/day, preferably yielding a Cmax of 1 nM to 1 µM.

More particularly, the amount administered should be in the range of 0.1-100 mg/day, preferably yielding a Cmax of 1 nM to 0.5 µM.

The HDAC inhibitors (HDACis) described herein may be administered to a subject in a convenient manner such as by the oral, intraveneous, intramuscular, subcutaneous, intraperitoneal, intranasal, buccal, transdermal, intradermal, or suppository routes as is known in the art. The active substances may also be administered to a human subject by continuous infusion over a predetermined time period, for example, from one minute up to 24 hours. Administration may be by way of an intravenous catheter connected to an appropriate pump, or by gravity feed.

The substances may be coated by, or administered with, a material to prevent its inactivation. For example, the active material may be administered in an adjuvant, co-administered with e.g. enzyme inhibitors or in liposomes. Adjuvants contemplated herein include, but are not limited to, resorcinols, non-ionic surfactants such as polyoxyethylene oleyl ether and n-hexadecyl polyethylene ether. Enzyme inhibitors include; but are not limited to, pancreatic trypsin inhibitor, diisopropylfluorophosphate (DFP) and trasylol. Liposymes include water-in-oil-in-water P40 emulsions as well as conventional liposomes. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include, but is not limited to, sterile aqueous solutions (where water soluble) or dispersions and sterile powders for the extemporaneous preparation of sterile injectable solutions or dispersion. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, sterile water, ethanol, polyol (for example, glycerol, propylene glycol and liquid polyethylene glycol and the like), suitable mixtures thereof, and vegetable oils. The proper fluidity can be maintained, for example, by the use of a coating such as lecithin, by the maintenance of the required particle size in the case of a dispersion, and by the use of surfactants. The preventions of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate, and gelatin.

Sterile injectable solutions are prepared by incorporating the active material in the required amount in the appropriate solvent with various of the other ingredients enumerated above, as required, followed by filtered sterilization. Generally, dispersions are prepared by incorporating the various sterilized active ingredient into a sterile vehicle which contains the basic dispersion medium and the required other ingredients from those enumerated above. In the case of sterile powders for the preparation of sterile injectable solutions, the preferred methods of preparation are vacuum drying and the freeze-drying technique, which yield a powder of the active ingredient plus any additional desired ingredient from previously sterile-filtered solution thereof.

When injected, higher plasma concentrations of HDACi may be temporarily achieved than is described above. However, the steady-state concentration lies within the concentrations mentioned in the application.

When the substances described herein are suitably protected as described above, the active compound may be orally administered, for example, with an inert diluent or with an edible carrier, or it may be enclosed in hard or soft shell gelatin capsule, or it may be compressed into tablets, or it may be incorporated directly with the food of the diet. For oral therapeutic administration, the active material may be incorporated with excipients and used in the form of ingestible tablets, buccal tablets, troches, capsules, elixirs, suspensions, syrups, wafers, and the like. In addition, the active material may be incorporated into sustained-release preparations and formulations. For example, the active material may be incorporated in enterotablets/capsules and/or bi-phasic release formulations, the latter described in e.g. US2007/0232528A1.

The tablets, troches, pills, capsules, and the like may also contain the following: A binder such as gum tragacanth, acacia, corn starch, or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavoring agent such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye, and flavoring such as cherry or orange flavor. Of course, any material used in preparing any dosage unit form should be pharamceutically pure and substantially non-toxic in the amounts employed.

In all administration forms and routes mentioned in the application, a mentioned HDACi substance or a pharmaceutically acceptable salt of this HDACi substance can be used. The invention covers the use of these HDACi substances as well as any known form of these substances, including but not limited to a pharmaceutically acceptable salt of the HDACi substances, in any suitable administration form or route known in the art.

Pharmaceutically acceptable salts of these compounds include but are not limited to:
(a) salts formed when an acidic proton is replaced by a metal ion, such as for example, an alkali metal ion (e.g. lithium, sodium, potassium), an alkaline earth ion (e.g. magnesium, or calcium), or an aluminum ion, or is replaced by an ammonium cation (NH₄⁺);
(b) salts formed by reacting the compound with a pharmaceutically acceptable organic base, which includes alkylamines, such as ethanolamine, diethanolamine, triethanolamine, tromethamine, N-methylglucamine, dicyclohexylamine, tris(hydroxymethyl)methylamine, and salts with amino acids such as arginine, lysine, and the like;
(c) salts formed by reacting the compound with a pharmaceutically acceptable acid, which provides acid addition salts. Pharmaceutically acceptable acids include hydrochloric acid, hydrobromic acid, sulfuric acid, nitric acid, phosphoric acid, metaphosphoric acid, and the like; or with an organic acid, such as, for example, acetic acid, propionic acid, hexanoic acid, cyclopentanepropionic acid, glycolic acid, pyruvic acid, lactic acid, malonic acid, succinic acid, malic acid, maleic acid, fumaric acid, trifluoroacetic acid, tartaric acid, citric acid, benzoic acid, 3-(4-hydroxybenzoyl)benzoic acid, cinnamic acid, mandelic acid, methanesulfonic acid, ethanesulfonic acid, 1,2-ethanedisulfonic acid, 2-hydroxyethanesulfonic acid, benzenesulfonic acid, toluenesulfonic acid, 2-naphthalenesulfonic acid, 4-methylbicyclo-[2.2.2]oct-2-ene-1-carboxylic acid, glucoheptonic acid, 4,4'-methylenebis-(3-hydroxy-2-ene-1-carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like.

Additional pharmaceutically acceptable salts include those described in Berge et al., J. Pharm. Sci. 1977, 66, 1-19; and "Handbook of Pharmaceutical Salts, Properties, and Use," Stah and Wermuth, Ed.; Wiley-VCH and VHCA, Zurich, 2002.

The compounds presented herein include all diastereomeric, enantiomeric, and epimeric forms. For compounds described herein that exist as tautomers, all tautomers are included within the formulas described herein. Further, the compounds described herein may be formed as, and/or used as, pharmaceutically acceptable salts.

Compounds described herein may be prepared using techniques and proceedures known to those skilled in the art. Exemplary synthetic methods useful for synthesizing the compounds in the application include, for example, those disclosed in Nogrady (1985) Medicinal Chemistry A Biochemical Approach, Oxford University Press, New York, pages 388-392; Silverman (1992); Fieser and Fieser's Reagents for Organic Synthesis, Volumes 1-17 (John Wiley and Sons, 1991); Rodd's Chemistry of Carbon Compounds, Volumes 1-5 and Supplementals (Elsevier Science Publishers, 1989); Organic Reactions, Volumes 1-40 (John Wiley and Sons, 1991), March's Advanced Organic Chemistry, (John Wiley and Sons, 4th Edition) and Larock's Comprehensive Organic Transformations (VCH Publishers Inc., 1989).

In partcular, compounds described herein may be commercially available and/or may be synthesised in accordance with published proceedures, as known to the skilled person and/or as mentioned herein. VPA may be commercially available from Sigma-Aldrich under product number P4543.

As used herein, the terms "pharmaceutically acceptable carrier" and "excipient" include any and all solvents, dispersion media, coatings, antibacterial and antifungal agents, isotonic and absorption delaying agents, and the like described above. The use of such carriers and excipients is well known in the art, see for example, Remington's Pharmaceutical Science and U.S. Pharmacopeia (The United States Pharmacopeia-National Formulary (USP-NF)), Remington: The Science and Practice of Pharmacy, 19th Ed. (Easton, Pa.: Mack Publishing Company, 1995); Hoover, John E., Remington's Pharmaceutical Sciences, Mack Publishing Co., Easton, Pa. 1975; Liberman, H. A. and Lachman, L., Eds., Pharmaceutical Dosage Forms, Marcel Decker, New York, N.Y., 1980; and Pharmaceutical Dosage Forms and Drug Delivery Systems, 7th Ed. (Lippincott Williams Wilkins 1999).

The skilled person will understand that the term "administered in combination with" includes concomitant and/or sequential administration. In this regard, sequential administration may involve administration within the same therapeutic intervention (e.g. within one hour of the compound of the invention).

In particular, the compound may be administered in association with one or more anticoagulant agents (i.e. an example of a class of drugs targeting clot formation), such as heparin, low molecular weight heparin (LMWH), warfarin, anisindione, phenindone, bishydroxycoumarin, bivalirudin, eptifibatid; and/or one or more vasodilators such as nitriles (for example, amylnitrile, nitroglycerin, sodium nitrile, isosorbide dinitrate), papaverine, nicotinic acid and cyclandelate. Anticoagulant and vasodilatory agents may improve access to thrombosis and other fibrin deposits thereby enhancing fibrin degradation.

More particularly, the active material may as well be administered in association with agents preventing cardiovascular events such as, but not limited to statins, beta blockers, angiotensin converting enzyme inhibitors, angiotensin II receptor antagonists or diuretics.

In particular, the compound may also be administered in association with one or more anti-inflammatory agents including steroids and NSAIDs (including but not limited to aspirin, ibuprofen, naproxen and diclofenac).

The active material may be administered in association with one or more anti-platelet agents (i.e. an example of a class of drugs targeting clot formation) including but not limited to aspirin, persantin and clopidogrel.

In particular disclosures, the other therapeutic agent is a drug targeting clot formation, such as one or more anti-platelet agents (e.g. aspirin, persantin and/or clopidogrel).

In more particular disclosures, the compound may also be administered in association with other HDACi substances, including but not limited to VPA and pharmaceutically acceptable salts of VPA.

For example, a combined treatment with VPA (using e.g. approximately 50-250 mg twice daily or a plasma concentration in the range of approximately 1 µM - 0.4 mM, preferably 1 µM - <0.35 mM) can make the treatment more effective and/or reduce the side effects. The active material may also be administered in association with one or more thrombolytic agents selected from, for example, recombinant t-PA, prourokinase, urokinase or streptokinase. Potentiation of fibrinolytic activity may take place when the HDACi is administered with such agents.

In particular disclosures, the compound is to be administered in association with VPA (for example, in a dose of VPA of approximately 50-250 mg twice daily and/or a dose that achieves a plasma concentration (e.g. a Cmax) in the range of approximately 1 µM - 0.4 mM, preferably 1 µM - <0.35 mM). In a further embodiment, the dose of VPA is as described in respect of the eleventh aspect of the invention (below)).

The disclosure herein is also concerned with thrombolytic compositions which comprise HDACi in association with one or more pharmaceutically acceptable carriers or excipients; and which optionally include one or more anti-thrombolytic agents, and/or one or more anticoagulant agents, and/or one or more antiplatelet agents and/or one or more vasodilators, as described above.

Whether the patient has a local or systemic inflammation that can be determined by identifying the presence of high sensitive C-reactive protein (hs-CRP) (at or above 2.0 mg/l serum) and/or fibrinogen (at or above 3g/l serum).

As discussed above, the invention makes it possible to use this treatment for preventing cardiovascular disease without the adverse side effects observed in other diseases treated with VPA (i.e. at higher concentrations).

Whether the patient has "endogenous fibrinolysis impaired by local or systemic inflammation" and/or "inflammation-suppressed fibrolytic function" as used herein can be determined using one or more biomarkers coupled to inflammation, including but not limited to C reactive protein, TNF-alpha, high sensitive C-reactive protein (hs-CRP), fibrinogen, IL-1beta, and IL-6 (e.g. by increased concentration of one or more of these biomarkers in relation to control levels as known in the art and as discussed herein (above)).

As used herein, the skilled person will understand that "prevention" may also be referred to as "prophylaxis".

The amounts of and dosage regimes of VPA which are administered to a subject to normalize or increase fibrinolysis will depend on a number of factors such as the mode of administration, the nature of the condition being treated, the body weight of the subject being treated, and the judgment of the prescribing physician. The VPA treatment can be given as a specific dose at a specific interval based on these factors. Alternatively, as there is a relatively high inter-individual variation in the plasma concentrations reached with a specific dose of VPA, the concentration of VPA in plasma can be continuously monitored and the patient titrated to reach a specific dose and interval that results in a desired plasma concentration. Generally speaking, VPA may be administered in an amount between 1 µg to 30 mg per kilogram of body weight per day. The concentration of VPA in plasma could be between 1 µM-2 mM. VPA may be administered to a subject in a once a week, bi-daily, daily, twice or thrice a day administration regimen in order to achieve the required steady state concentration of the substance in plasma. Preferably, the amount administered should be in the range of approximately 50-1000 mg/day and plasma concentrations reach approximately 0.01-0.7 mM. More preferably, the amount administered should be approximately 50-250 mg twice daily and the plasma concentration should be in the range of approximately 0.05- 0.4 mM. Even more preferably, the amount administered should be approximately 50-200 mg twice daily and the plasma concentration should be in the range of approximately 0.05-0.35 mM. Most preferably, the amount administered results in a plasma concentration in the range of approximately 0.05-0.3 mM. In a preferred embodiment of the invention, VPA will be administered twice daily to yield a plasma concentration below 0.3 mM (such as 0.05-0.29 mM).

In particular disclosures, the valproic acid, or a pharmaceutically acceptable salt thereof, is administered in an amount between 1 µg to 30 mg per kilogram of body weight per day, preferably yielding a Cmax in the range of approximately 1 µM-2 mM.

In particular disclosures, the amount of valproic acid, or a pharmaceutically acceptable salt thereof, administered should be in the range of approximately 50-1000 mg/day, preferably yielding a Cmax in the range of approximately 0.01-0.7 mM. In a more particular embodiment, the amount administered should be approximately 50-250 mg twice daily, preferably yielding a Cmax in the range of approximately 0.05- 0.4 mM. In a further embodiment, the amount administered should be approximately 50-200 mg twice daily, preferably yielding a Cmax in the range of approximately 0.05-0.35 mM.

In particular disclosures, the amount of valproic acid, or a pharmaceutically acceptable salt thereof, administered results in a plasma concentration in the range of approximately 0.05-0.3 mM. In a preferred embodiment of the invention, valproic acid, or a pharmaceutically acceptable salt thereof, will be administered twice daily to yield a plasma concentration below 0.3 mM (such as 0.01-0.29 mM).

When injected, higher plasma concentrations of VPA than is described above may be temporarily achieved. However, the steady-state concentration lies within the previously described concentrations.

Valproic acid, or a pharmaceutically acceptable salt thereof, of this application may be administered to a subject in a convenient manner such those manners described in respect of HDAC inhibitors (HDACis) above.

In all administration forms and routes mentioned in the application, VPA or a pharmaceutically acceptable salt of VPA can be used. The invention covers the use of VPA as well as any form of VPA known in the art, including but not limited to pharmaceutically acceptable salts of VPA in any suitable administation form or route known in the art.

Pharmaceutically acceptable salts of VPA include but are not limited to:
(a) salts formed when an acidic proton is replaced by a metal ion, such as for example, an alkali metal ion (e.g. lithium, sodium, potassium), an alkaline earth ion (e.g. magnesium, or calcium), or an aluminum ion, or is replaced by an ammonium cation (NH₄⁺);
(b) salts formed by reacting VPA with a pharmaceutically acceptable organic base, which includes alkylamines, such as ethanolamine, diethanolamine, triethanolamine, tromethamine, N-methylglucamine, dicyclohexylamine, tris(hydroxymethyl)methylamine, and salts with amino acids such as arginine, lysine, and the like;
(c) salts formed by reacting VPA with a pharmaceutically acceptable acid, which provides acid addition salts. Pharmaceutically acceptable acids include hydrochloric acid, hydrobromic acid, sulfuric acid, nitric acid, phosphoric acid, metaphosphoric acid, and the like; or with an organic acid, such as, for example, acetic acid, propionic acid, hexanoic acid, cyclopentanepropionic acid, glycolic acid, pyruvic acid, lactic acid, malonic acid, succinic acid, malic acid, maleic acid, fumaric acid, trifluoroacetic acid, tartaric acid, citric acid, benzoic acid, 3-(4-hydroxybenzoyl)benzoic acid, cinnamic acid, mandelic acid, methanesulfonic acid, ethanesulfonic acid, 1,2-ethanedisulfonic acid, 2-hydroxyethanesulfonic acid, benzenesulfonic acid, toluenesulfonic acid, 2-naphthalenesulfonic acid, 4-methylbicyclo-[2.2.2]oct-2-ene-1-carboxylic acid, glucoheptonic acid, 4,4'-methylenebis-(3-hydroxy-2-ene-1-carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like.

Additional pharmaceutically acceptable salts include those described in Berge et al., J. Pharm. Sci. 1977, 66, 1-19; and "Handbook of Pharmaceutical Salts, Properties, and Use," Stah and Wermuth, Ed.; Wiley-VCH and VHCA, Zurich, 2002.

Valproic acid, or a pharmaceutically acceptable salt thereof, may be administered in association with one or more pharmaceutically acceptable carriers or excipients and one or more drugs targeting the formation of the clot.

Also described herein, there is provided a method, compound for use or use wherein valproic acid, or a pharmaceutically acceptable salt thereof, is administered in association with one or more pharmaceutically acceptable carriers or excipients and one or more drugs targeting the formation of the clot.

Valproic acid (or a pharmaceutically acceptable salt thereof) may administered in association with one or more anti-platelet agents including but not limited to aspirin, persantin and clopidogrel. It may also be administered in association with one or more anticoagulant agents, such as heparin, low molecular weight heparin (LMWH), warfarin, anisindione, phenindone, bishydroxycoumarin, bivalirudin, eptifibatid; and/or one or more vasodilators such as nitriles (for example, amylnitrile, nitroglycerin, sodium nitrile, isosorbide dinitrate), papaverine, nicotinic acid and cyclandelate. Anticoagulant and vasodilatory agents may improve access to thrombosis and other fibrin deposits thereby enhancing fibrin degradation. Further, valproic acid (or a pharmaceutically acceptable salt thereof) may as well be administered in association with agents preventing cardiovascular events such as, but not limited to statins, beta blockers, angiotensin converting enzyme inhibitors, angiotensin II receptor antagonists or diuretics. Valproic acid (or a pharmaceutically acceptable salt thereof) may also be administered in association with one or more anti-inflammatory agents including steroids and NSAIDs (including but not limited to aspirin, ibuprofen, naproxen and diclofenac). Valproic acid (or a pharmaceutically acceptable salt thereof) may also be administered in association with one or more thrombolytic agents selected from, for example, recombinant tPA, prourokinase, urokinase or streptokinase. Without wishing to be bound by theory, potentiation of fibrinolytic activity may take place when VPA is administered with such agents.

In a particular disclosure, valproic acid (or a pharmaceutically acceptable salt thereof) is administered in association with one or more anti-platelet agents including but not limited to aspirin, persantin and clopidogrel.

In another disclosure, valproic acid (or a pharmaceutically acceptable salt thereof) is administered with one or more anticoagulant agents, such as heparin, low molecular weight heparin (LMWH), warfarin, anisindione, phenindone, bishydroxycoumarin, bivalirudin, eptifibatid; and/or one or more vasodilators such as nitriles (for example, amylnitrile, nitroglycerin, sodium nitrile, isosorbide dinitrate), papaverine, nicotinic acid and cyclandelate.

In another disclosure, valproic acid (or a pharmaceutically acceptable salt thereof) is administered in association with agents preventing cardiovascular events such as, but not limited to statins, beta blockers, angiotensin converting enzyme inhibitors, angiotensin II receptor antagonists or diuretics.

In another disclosure, valproic acid (or a pharmaceutically acceptable salt thereof) is administered in association with one or more anti-inflammatory agents including steroids and NSAIDs (including but not limited to aspirin, ibuprofen, naproxen and diclofenac).

In another disclosure, valproic acid (or a pharmaceutically acceptable salt thereof) is administered in association with one or more thrombolytic agents selected from, for example, recombinant tPA, prourokinase, urokinase or streptokinase.

Also described herein are thrombolytic compositions which comprise VPA in association with one or more pharmaceutically acceptable carriers or excipients; and which optionally include one or more anti-thrombolytic agents, and/or one or more anticoagulant agents, and/or one or more antiplatelet agents and/or one or more vasodilators, as described above.

Thus, also described herein, there is provided a pharmaceutical composition comprising:
(a) valproic acid, or a pharmaceutically acceptable salt thereof;
(b) one or more pharmaceutically acceptable carriers or excipients; and
(c) one or more anti-thrombolytic agents, and/or one or more anticoagulant agents, and/or one or more antiplatelet agents and/or one or more vasodilators,
wherein the anti-thrombolytic agents, anticoagulant agents, antiplatelet agents and vasodilators are as described in respect of the eleventh aspect of the invention.

In a particular disclosure, valproic acid, or a pharmaceutically acceptable salt thereof, is present in a dose as defined herein.

Also described herein, there is provided a kit of parts comprising:
(A) valproic acid, or a pharmaceutically acceptable salt thereof;
(B) one or more pharmaceutically acceptable carriers or excipients; and
(C) one or more anti-thrombolytic agents, and/or one or more anticoagulant agents, and/or one or more antiplatelet agents and/or one or more vasodilators,
wherein the anti-thrombolytic agents, anticoagulant agents, antiplatelet agents and vasodilators are as described in respect of the thirteenth aspect of the invention.

For the avoidance of doubt, it is specifically intended that references to other (e.g. preceding) aspects include a reference to each embodiment (e.g. particular or preferred embodiments) of that aspect and combinations thereof.

### EXAMPLES

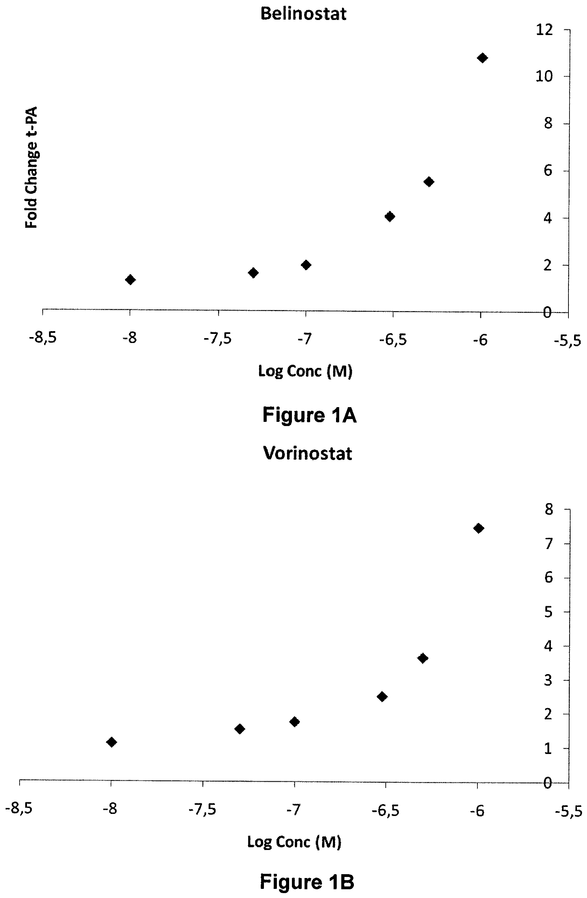

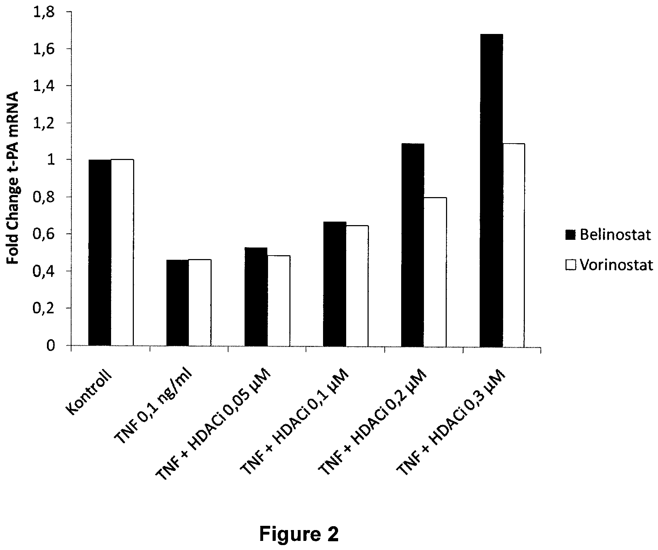

The following Examples and Refernce Example further illustrate the invention and other disclosures provided herein. It will, of course, be understood that the invention is in no way restricted to the specific aspects described in these Examples.
REFERENCE EXAMPLE 1
*In vitro* dose response experiment for Vorinostat
Human umbilical vein endothelial cells (HUVECs) were prepared by collagenase treatment of fresh umbilical cords (Jaffe, E.A., et al. J Clin Invest 52, 2745-2756 (1973)) obtained from the maternity ward of the Sahlgrenska University hospital, Gotheburg, Sweden. Cells were cultured in EGM-2 medium (Lonza, Basel, Switzerland) and all experiments were performed in passage 1 of subcultivation. Confluent HUVECs were exposed to 10 nM-10 µM of Vorinostat (Selleck Chemicals, Houston, TX, USA) in complete medium for 24 h. After 24 h, cells and conditioned media were harvested. Total RNA was prepared using RNeasy Mini RNA kit (Qiagen, Hilden, Germany) and genomic DNA was removed using RNase-free DNase I set (Qiagen). Levels of t-PA mRNA were analyzed with real-time RT-PCR, performed on an Applied Biosystems 7500 Fast Real-Time PCR System using cDNA and Taqman reagents obtained from Applied Biosystems (Foster City, CA, USA). Hypoxanthine phosphoribosyl transferase (HPRT, Assay number Hs99999909_m1, Applied Biosystems) was used as endogenous internal standard.
Endothelial cells in culture are known to constitutively secrete the majority of synthesized t-PA making conditioned media a suitable source for quantification of t-PA protein. Conditioned medium from cell cultures was collected, centrifuged (10 000 x g, 10 min, 4 °C) to remove cell debris, transferred to fresh tubes and stored at -70 °C. Concentrations of t-PA antigen in conditioned media were determined using the commercially available TriniLize t-PA antigen ELISA (Trinity Biotech, Bray, Ireland) according to manufacturer's protocol.
A significant increase of t-PA mRNA and protein levels could be seen already at 50 nM of Vorinostat. The effect on t-PA expression was increased in a dose-dependent manner and maximal at around 3 µM where t-PA expression was increased approximately 7 times (Figure 1 B).
REFERENCE EXAMPLE 2
*In vitro* dose response experiment for Belinostat
Belinostat was studied according to the protocol described in Example 1. Cells were treated with 10 nM-10 µM of Belinostat (Selleck Chemicals, Houston, TX, USA) for 24 h. A significant increase of t-PA mRNA levels could be seen already at 10 nM of Belinostat. The effect on t-PA expression was increased in a dose-dependent manner and maximal at around 3 µM where t-PA expression was increased approximately 10 times (Figure 1).
REFERENCE EXAMPLE 3
*In vitro* dose response experiment for Givinostat
Givinostat is studied according to the protocol described in Example 1. Cells are treated with 1 nM-10 µM of Givinostat for 24 h.
A significant increase of t-PA mRNA levels is seen already at 10 nM of Givinostat (Selleck Chemicals, Houston, TX, USA). The effect on t-PA expression is increased in a dose-dependent manner and maximal at around 0.3 µM where t-PA expression is increased approximately 10 times.
REFERENCE EXAMPLE 4
*In vitro* dose response experiment for Panobinostat
Panobinostat is studied according to the protocol described in Example 1. Cells are treated with 0.1 nM-10 µM of Panobinostat (Selleck Chemicals, Houston, TX, USA) for 24 h.
A significant increase of t-PA mRNA levels is seen already at 1 nM of Panobinostat. The effect on t-PA expression is increased in a dose-dependent manner and maximal at around 30 nM where t-PA expression is increased approximately 10 times.
REFERENCE EXAMPLE 5
*In vitro* dose response experiment for PCI-24781
PCI-24781 is studied according to the protocol described in Example 1. Cells are treated with 1 nM-10 µM of PCI-24781 (Selleck Chemicals, Houston, TX, USA) for 24 h.
A significant increase of t-PA mRNA levels is seen already at 1 nM of PCI-24781. The effect on t-PA expression is increased in a dose-dependent manner and maximal at around 0.3 µM where t-PA expression is increased approximately 10 times.
REFERENCE EXAMPLE 6
*In vitro* dose response experiment for JNJ-26481585
JNJ-26481585 is studied according to the protocol described in Example 1. Cells are treated with 0.1 nM-1 µM of JNJ-26481585 (Selleck Chemicals, Houston, TX, USA) for 24 h.
A significant increase of t-PA mRNA levels is seen already at 1 nM of JNJ-26481585. The effect on t-PA expression is increased in a dose-dependent manner and maximal at around 30 nM where t-PA expression is increased approximately 10 times.
REFERENCE EXAMPLE 7
*In vitro* dose response experiment for Mocetinostat
Mocetinostat is studied according to the protocol described in Example 1. Cells are treated with 10 nM-10 µM of Mocetinostat (Selleck Chemicals, Houston, TX, USA) for 24 h.
A significant increase of t-PA mRNA levels is seen already at 10 nM of Mocetinostat. The effect on t-PA expression is increased in a dose-dependent manner and maximal at around 3 µM where t-PA expression is increased approximately 10 times.
REFERENCE EXAMPLE 8
*In vitro* dose response experiment for SB939
SB939 is studied according to the protocol described in Example 1. Cells are treated with 10 nM-10 µM of SB939 (Selleck Chemicals, Houston, TX, USA) for 24 h.
A significant increase of t-PA mRNA levels is seen already at 10 nM of SB939. The effect on t-PA expression is increased in a dose-dependent manner and maximal at around 1 µM where t-PA expression is increased approximately 10 times.
REFERENCE EXAMPLE 9
*In vitro* dose response experiment for CXD101
CXD101 is studied according to the protocol described in Example 1. Cells are treated with 1 nM-10 µM of CXD101 (Celleron Therapeutics, Oxon, UK) for 24 h.
A significant increase of t-PA mRNA levels is seen already at 10 nM of CXD101. The effect on t-PA expression is increased in a dose-dependent manner and maximal at around 3 µM where t-PA expression is increased approximately 10 times.
REFERENCE EXAMPLE 10
Counter-acting inflammatory suppression of t-PA with Belinostat
We have previously shown that proinflammatory cytokines e.g. TNF-alpha and IL-1 b suppress t-PA production in endothelial cells. We wanted to determine the capacity of Belinostat to reverse such a TNF-alpha suppressed t-PA response in HUVECs.
Human umbilical vein endothelial cells (HUVECs) were prepared and cultured as described in Example 1. Confluent HUVECs were exposed to low concentrations of TNF-alpha (0.1 ng/ml) (Sigma-Aldrich) for 24 h. Thereafter, medium was replaced by fresh EGM-2 containing TNF-alpha and low concentrations of belinostat (10 nM to 300 nM) and incubated for 24 h. After 24 h, cells and conditioned media were harvested. Total RNA was prepared and RNA and secreted protein quantified as in Example 1. Prolonged stimulation (48 h) with 0.1 ng/ml of TNF-alpha caused a significant 2-fold suppression of t-PA production. This could be partly reversed with as low as 50 nM and completely normalized with 200 nM of Belinostat (Figure 2).
REFERENCE EXAMPLE 11
Counter-acting inflammatory suppression of t-PA with Vorinostat
Vorinostat was studied according to the protocol described in Example 10. After an initial 24 h TNF stimulation, cells were treated with 10 nM to 300 nM Vorinostat for 24 h. Prolonged stimulation (48 h) with 0.1 ng/ml of TNF-alpha caused a significant 2-fold suppression of t-PA production. This could be partly reversed with as low as 50 nM and completely normalized with 300 nM of Vorinostat (Figure 2).
REFERENCE EXAMPLE 12
Counter-acting inflammatory suppression of t-PA with Givinostat
Givinostat is studied according to the protocol described in Example 10. After an initial 24 h TNF stimulation, cells are treated with 10 nM to 300 nM Givinostat for 24 h. Prolonged stimulation (48 h) with 0.1 ng/ml of TNF-alpha caused a significant 2-fold suppression of t-PA production. This is partly reversed with as low as 30 nM and completely normalized with 100 nM of Givinostat.
REFERENCE EXAMPLE 13
Counter-acting inflammatory suppression of t-PA with Panobinostat
Panobinostat is studied according to the protocol described in Example 10. After an initial 24 h TNF stimulation, cells are treated with 1 nM to 300 nM Panobinostat for 24 h. Prolonged stimulation (48 h) with 0.1 ng/ml of TNF-alpha caused a significant 2-fold suppression of t-PA production. This is partly reversed with as low as 1 nM and completely normalized with 5 nM of Panobinostat.
REFERENCE EXAMPLE 14
Counter-acting inflammatory suppression of t-PA with PCI-24781
PCI-24781 is studied according to the protocol described in Example 10. After an initial 24 h TNF stimulation, cells are treated with 10 nM to 300 nM PCI-24781 for 24 h. Prolonged stimulation (48 h) with 0.1 ng/ml of TNF-alpha caused a significant 2-fold suppression of t-PA production. This is partly reversed with as low as 10 nM and completely normalized with 200 nM of PCI-24781.
REFERENCE EXAMPLE 15
Counter-acting inflammatory suppression of t-PA with JNJ-26481585
JNJ-26481585 is studied according to the protocol described in Example 10. After an initial 24 h TNF stimulation, cells are treated with 1 nM to 300 nM JNJ-26481585 for 24 h.
Prolonged stimulation (48 h) with 0.1 ng/ml of TNF-alpha caused a significant 2-fold suppression of t-PA production. This is partly reversed with as low as 1 nM and completely normalized with 5 nM of JNJ-26481585.
REFERENCE EXAMPLE 16
Counter-acting inflammatory suppression of t-PA with Mocetinostat
Mocetinostat is studied according to the protocol described in Example 10. After an initial 24 h TNF stimulation, cells are treated with 10 nM to 300 nM Mocetinostat for 24 h. Prolonged stimulation (48 h) with 0.1 ng/ml of TNF-alpha caused a significant 2-fold suppression of t-PA production. This is partly reversed with as low as 10 nM and completely normalized with 300 nM of Mocetinostat.
REFERENCE EXAMPLE 17
Counter-acting inflammatory suppression of t-PA with SB939
SB939 is studied according to the protocol described in Example 10. After an initial 24 h TNF stimulation, cells are treated with 10 nM to 300 nM SB939 for 24 h.
Prolonged stimulation (48 h) with 0.1 ng/ml of TNF-alpha caused a significant 2-fold suppression of t-PA production. This is partly reversed with as low as 10 nM and completely normalized with 300 nM of SB939.
REFERENCE EXAMPLE 18
Counter-acting inflammatory suppression of t-PA with CXD101
CXD101 is studied according to the protocol described in Example 10. After an initial 24 h TNF stimulation, cells are treated with 10 nM to 300 nM CXD101 for 24 h.
Prolonged stimulation (48 h) with 0.1 ng/ml of TNF-alpha caused a significant 2-fold suppression of t-PA production. This is partly reversed with as low as 10 nM and completely normalized with 300 nM of CXD101.
REFERENCE EXAMPLE 19
Intermediate endpoint study: Effects of Vorinostat on *in vivo* t-PA release in man
An intermediate endpoint proof-of-concept study is performed in patients with atherosclerotic disease investigated before and after treatment with Vorinostat.
The study comprises 16 patients with stable angina pectoris. Patients are investigated before and after oral treatment with 10 mg Vorinostat (Zolinza®, Merck & Co., Inc, NJ, USA) daily for 2 weeks. The study has a randomized, cross-over design and t-PA release capacity is investigated before and after treatment, with each individual serving as his/her own control.
The capacity for t-PA release is investigated in the perfused-forearm model that we have developed, which is the only method that permits a direct measurement of the local release of t-PA from the endothelium (Hrafnkelsdottir, T., et al. Lancet 352, 1597-1598 (1998), Wall, U., et al. Blood 91, 529-537 (1998)). Since t-PA has a rapid hepatic clearance, it is impossible to infer endothelial release rates from plasma levels obtained from standard venous samples. With the invasive model, however, net forearm t-PA release rates are calculated from arterio-venous concentration gradients of t-PA after correction for forearm plasma flow. Acute t-PA release responses are induced by intraarterial infusions of Substance P (Bachem, Bubendorf, Switzerland), and the amount and protein secretion profile is used as a measure of t-PA release capacity.
Comparison of the t-PA secretion profiles before and after treatment with Vorinostat shows that the total amount of t-PA released area under the curve (AUC) is increased by approximately 50%. This study shows that there is a significant improvement of the cumulative amount of t-PA released across the forearm vasculature in response to the stimulation after short-term treatment with Vorinostat.
REFERENCE EXAMPLE 20
Intermediate endpoint study: Effects of Belinostat on *in vivo* t-PA release in man Belinostat is studied according to the same protocol as in Example 19. Patients are treated with 65 mg Belinostat (TopoTarget, Copenhagen, Denmark) daily for 2 weeks. Comparison of the t-PA secretion profiles before and after treatment with Belinostat shows that the total amount of t-PA released (AUC) is increased by approximately 50%. This study shows that there is a significant improvement of the cumulative amount of t-PA released across the forearm vasculature in response to the stimulation after short-term treatment with Belinostat.
REFERENCE EXAMPLE 21
Intermediate endpoint study: Effects of Givinostat on *in vivo* t-PA release in man Givinostat is studied according to the same protocol as in Example 19. Patients are treated with 2 mg Givinostat (Italfarmaco, Milan, Italy) daily for 2 weeks.
Comparison of the t-PA secretion profiles before and after treatment with Givinostat shows that the total amount of t-PA released (AUC) is increased by approximately 50%. This study shows that there is a significant improvement of the cumulative amount of t-PA released across the forearm vasculature in response to the stimulation after short-term treatment with Givinostat.
REFERENCE EXAMPLE 22
Intermediate endpoint study: Effects of Panobinostat on *in vivo* t-PA release in man Panobinostat is studied according to the same protocol as in Example 19. Patients are treated with 0.5 mg Panobinostat (Novartis, Cambridge, MA, USA) daily for 2 weeks. Comparison of the t-PA secretion profiles before and after treatment with Panobinostat shows that the total amount of t-PA released (AUC) is increased by approximately 50%. This study shows that there is a significant improvement of the cumulative amount of t-PA released across the forearm vasculature in response to the stimulation after short-term treatment with Panobinostat.
REFERENCE EXAMPLE 23
Intermediate endpoint study: Effects of PCI-24781 on *in vivo* t-PA release in man PCI-24781 is studied according to the same protocol as in Example 19. Patients are treated with 2mg PCI-24781 (Pharmacyclics, Sunnyvale, CA, USA) daily for 2 weeks. Comparison of the t-PA secretion profiles before and after treatment with PCI-24781 shows that the total amount of t-PA released (AUC) is increased by approximately 50%. This study shows that there is a significant improvement of the cumulative amount of t-PA released across the forearm vasculature in response to the stimulation after short-term treatment with PCI-24781.
REFERENCE EXAMPLE 24
Intermediate endpoint study: Effects of JNJ-26481585 on *in vivo* t-PA release in man JNJ-26481585 is studied according to the same protocol as in Example 19. Patients are treated with 0.2 mg JNJ-26481585 (Johnson&Johnson Pharmaceutical Research and Development, La Jolla, CA, USA) daily for 2 weeks.
Comparison of the t-PA secretion profiles before and after treatment with JNJ-26481585 shows that the total amount of t-PA released (AUC) is increased by approximately 50%. This study shows that there is a significant improvement of the cumulative amount of t-PA released across the forearm vasculature in response to the stimulation after short-term treatment with JNJ-26481585.
REFERENCE EXAMPLE 25
Intermediate endpoint study: Effects of Mocetinostat on *in vivo* t-PA release in man Mocetinostat is studied according to the same protocol as in Example 19. Patients are treated with 2 mg of Mocetinostat (Methylgene, Montreal, Canada) daily for 2 weeks. Comparison of the t-PA secretion profiles before and after treatment with Mocetinostat shows that the total amount of t-PA released (AUC) is increased by approximately 50%. This study shows that there is a significant improvement of the cumulative amount of t-PA released across the forearm vasculature in response to the stimulation after short-term treatment with Mocetinostat.
REFERENCE EXAMPLE 26
Intermediate endpoint study: Effects of SB939 on *in vivo* t-PA release in man
SB939 is studied according to the same protocol as in Example 19. Patients are treated with 0.4 mg SB939 (S*BIO, Singapore) daily for 2 weeks.
Comparison of the t-PA secretion profiles before and after treatment with SB939 shows that the total amount of t-PA released (AUC) is increased by approximately 50%. This study shows that there is a significant improvement of the cumulative amount of t-PA released across the forearm vasculature in response to the stimulation after short-term treatment with SB939.
REFERENCE EXAMPLE 27
Intermediate endpoint study: Effects of CXD101 on *in vivo* t-PA release in man
CXD101 is studied according to the same protocol as in Example 19. Patients are treated with 10 mg CXD101 (Celleron Theraputics, Oxon, UK) daily for 2 weeks.
Comparison of the t-PA secretion profiles before and after treatment with CXD101 shows that the total amount of t-PA released (AUC) is increased by approximately 50%. This study shows that there is a significant improvement of the cumulative amount of t-PA released across the forearm vasculature in response to the stimulation after short-term treatment with CXD101.
EXAMPLE 28
Clinical outcome study in high-risk patients for prevention of recurrent events using Vorinostat
The first clinical outcome study is performed in high-risk patients who have experienced a recent major atherothrombotic cardiovascular event (myocardial infarction or ischemic stroke) to investigate the preventive effect of Vorinostat treatment on the risk for recurrent events. The annual risk for a recurrent atherothrombotic event in the investigated population is estimated to approximately 7%. Patients are randomized in a parallel study design to receive double-blind oral treatment with 10 mg Vorinostat or placebo daily, in addition to optimal conventional treatment. The event rate is monitored by Kaplan-Meyer statistics. The primary efficacy endpoint is the composite measure of either mortality, or non-fatal myocardial infarction or ischemic stroke. The study is event-driven to a total of 180 events in the placebo group.
The study shows that long-term Vorinostat treatment reduces this risk by approximately 30% in addition to that of conventional therapy, i.e. lowers the annual absolute event rate to approximately 5%. Thus, this study confirms the clinical efficacy and feasibility of using Vorinostat for secondary prevention of cardiovascular events.
REFERENCE EXAMPLE 29
Clinical outcome study in high-risk patients for prevention of recurrent events using Belinostat
Belinostat is studied according to the same protocol as in Example 28. Patients are randomized to 65 mg Belinostat or placebo daily.
The study shows that long-term Belinostat treatment reduces this risk by approximately 30% in addition to that of conventional therapy, i.e. lowers the annual absolute event rate to approximately 5%. Thus, this study confirms the clinical efficacy and feasibility of using Belinostat for secondary prevention of cardiovascular events.
REFERENCE EXAMPLE 30
Clinical outcome study in high-risk patients for prevention of recurrent events using Givinostat
Givinostat is studied according to the same protocol as in Example 28. Patients are randomized to 2 mg Givinostat or placebo daily.
The study shows that long-term Givinostat treatment reduces this risk by approximately 30% in addition to that of conventional therapy, i.e. lowers the annual absolute event rate to approximately 5%. Thus, this study confirms the clinical efficacy and feasibility of using Givinostat for secondary prevention of cardiovascular events.
REFERENCE EXAMPLE 31
Clinical outcome study in high-risk patients for prevention of recurrent events using Panobinostat
Panobinostat is studied according to the same protocol as in Example 28. Patients are randomized to 0.5 mg Panobinostat or placebo daily.
The study shows that long-term Panobinostat treatment reduces this risk by approximately 30% in addition to that of conventional therapy, i.e. lowers the annual absolute event rate to approximately 5%. Thus, this study confirms the clinical efficacy and feasibility of using Panobinostat for secondary prevention of cardiovascular events.
REFERENCE EXAMPLE 32
Clinical outcome study in high-risk patients for prevention of recurrent events using PCI-24781
PCI-24781 is studied according to the same protocol as in Example 28. Patients are randomized 2 mg PCI-24781 or placebo daily.
The study shows that long-term PCI-24781 treatment reduces this risk by approximately 30% in addition to that of conventional therapy, i.e. lowers the annual absolute event rate to approximately 5%. Thus, this study confirms the clinical efficacy and feasibility of using PCI-24781 for secondary prevention of cardiovascular events.
REFERENCE EXAMPLE 33
Clinical outcome study in high-risk patients for prevention of recurrent events using JNJ-26481585
JNJ-26481585 is studied according to the same protocol as in Example 28. Patients are randomized 0.2 mg JNJ-26481585 or placebo daily.
The study shows that long-term JNJ-26481585 treatment reduces this risk by approximately 30% in addition to that of conventional therapy, i.e. lowers the annual absolute event rate to approximately 5%. Thus, this study confirms the clinical efficacy and feasibility of using JNJ-26481585 for secondary prevention of cardiovascular events.
REFERENCE EXAMPLE 34
Clinical outcome study in high-risk patients for prevention of recurrent events using Mocetinostat
Mocetinostat is studied according to the same protocol as in Example 28. Patients are randomized to 2mg Mocetinostat or placebo daily.
The study shows that long-term Mocetinostat treatment reduces this risk by approximately 30% in addition to that of conventional therapy, i.e. lowers the annual absolute event rate to approximately 5%. Thus, this study confirms the clinical efficacy and feasibility of using Mocetinostat for secondary prevention of cardiovascular events.
REFERENCE EXAMPLE 35
Clinical outcome study in high-risk patients for prevention of recurrent events using SB939
SB939 is studied according to the same protocol as in Example 28. Patients are randomized to 0.4 mg SB939 or placebo daily.
The study shows that long-term SB939 treatment reduces this risk by approximately 30% in addition to that of conventional therapy, i.e. lowers the annual absolute event rate to approximately 5%. Thus, this study confirms the clinical efficacy and feasibility of using SB939 for secondary prevention of cardiovascular events.
REFERENCE EXAMPLE 36
Clinical outcome study in high-risk patients for prevention of recurrent events using CXD101
CXD101 is studied according to the same protocol as in Example 28. Patients are randomized to 10 mg CXD101 or placebo daily.
The study shows that long-term CXD101 treatment reduces this risk by approximately 30% in addition to that of conventional therapy, i.e. lowers the annual absolute event rate to approximately 5%. Thus, this study confirms the clinical efficacy and feasibility of using CXD101 for secondary prevention of cardiovascular events.
REFERENCE EXAMPLE 37
Clinical outcome study in unstable angina/non-ST segment elevation myocardial infarction using Vorinostat
The second clinical outcome study is performed in patients with non-ST-segment elevation acute coronary syndromes. This study is a randomized, double-blind trial enrolling approximately 7,000 patients within 72 hours of presentation with either unstable angina or non-ST segment elevation myocardial infarction who are not intended to undergo revascularization procedures for their index event. Patients are randomly allocated to Vorinostat or placebo treatment for a median duration of 18 months, in addition to standard medical therapy. In-hospital treatment is initiated as an intravenous infusion of Vorinosat followed by oral treatment with 10 mg Vorinostat daily. The primary composite efficacy endpoint will be time to first occurrence of cardiovascular death, new non-fatal myocardial infarction, non-fatal stroke, or severe myocardial ischemia requiring urgent revascularization. The treatment shows that Vorinostat can effectively reduce the risk for future major cardiovascular events. The risk is reduced by approximately 30% in addition to that of conventional therapy, i.e. lowers the annual absolute event rate to approximately 5%. Thus, this study confirms the clinical efficacy and feasibility of using Vorinostat for secondary prevention of cardiovascular events.
REFERENCE EXAMPLE 38
Clinical outcome study in unstable angina/non-ST segment elevation myocardial infarction using Belinostat
Belinostat is studied according to the same protocol as in Example 37. Patients are randomly allocated to Belinostat or placebo treatment for a median duration of 18 months. In- hospital treatment is initiated as an intravenous infusion of Belinostat followed by oral treatment with 65 mg Belinostat daily.
The treatment shows that Belinostat can effectively reduce the risk for future major cardiovascular events. The risk is reduced by approximately 30% in addition to that of conventional therapy, i.e. lowers the annual absolute event rate to approximately 5%. Thus, this study confirms the clinical efficacy and feasibility of using Belinostat for secondary prevention of cardiovascular events.
REFERENCE EXAMPLE 39
Clinical outcome study in unstable angina/non-ST segment elevation myocardial infarction using Givinostat
Givinostat is studied according to the same protocol as in Example 37. Patients are randomly allocated to Givinostat or placebo treatment for a median duration of 18 months. In- hospital treatment is initiated as an intravenous infusion of Givinostat followed by oral treatment with 2 mg Givinostat daily.
The treatment shows that Givinostat can effectively reduce the risk for future major cardiovascular events. The risk is reduced by approximately 30% in addition to that of conventional therapy, i.e. lowers the annual absolute event rate to approximately 5%. Thus, this study confirms the clinical efficacy and feasibility of using Givinostat for secondary prevention of cardiovascular events.
REFERENCE EXAMPLE 40
Clinical outcome study in unstable angina/non-ST segment elevation myocardial infarction using Panobinostat
Panobinostat is studied according to the same protocol as in Example 37. Patients are randomly allocated to Panobinostat or placebo treatment for a median duration of 18 months. In- hospital treatment is initiated as an intravenous infusion of Panobinostat followed by oral treatment with 0.5 mg Panobinostat daily.
The treatment shows that Panobinostat can effectively reduce the risk for future major cardiovascular events. The risk is reduced by approximately 30% in addition to that of conventional therapy, i.e. lowers the annual absolute event rate to approximately 5%. Thus, this study confirms the clinical efficacy and feasibility of using Panobinostat for secondary prevention of cardiovascular events.
REFERENCE EXAMPLE 41
Clinical outcome study in unstable angina/non-ST segment elevation myocardial infarction using PCI-24741
PCI-24781 is studied according to the same protocol as in Example 37. Patients are randomly allocated to PCI-24781 or placebo treatment for a median duration of 18 months. In- hospital treatment is initiated as an intravenous infusion of PCI-24781 followed by oral treatment with 2 mg PCI-24781 daily.
The treatment shows that PCI-24781 can effectively reduce the risk for future major cardiovascular events. The risk is reduced by approximately 30% in addition to that of conventional therapy, i.e. lowers the annual absolute event rate to approximately 5%. Thus, this study confirms the clinical efficacy and feasibility of using PCI-24781 for secondary prevention of cardiovascular events.
EXAMPLE 42
Clinical outcome study in unstable angina/non-ST segment elevation myocardial infarction using JNJ-26481585
JNJ-26481585 is studied according to the same protocol as in Example 37. Patients are randomly allocated to JNJ-26481585 or placebo treatment for a median duration of 18 months. In-hospital treatment is initiated as an intravenous infusion of JNJ-26481585 followed by oral treatment with 0.2 mg JNJ-26481585 daily.
The treatment shows that JNJ-26481585 can effectively reduce the risk for future major cardiovascular events. The risk is reduced by approximately 30% in addition to that of conventional therapy, i.e. lowers the annual absolute event rate to approximately 5%. Thus, this study confirms the clinical efficacy and feasibility of using JNJ-26481585 for secondary prevention of cardiovascular events.
REFERENCE EXAMPLE 43
Clinical outcome study in unstable angina/non-ST segment elevation myocardial infarction using Mocetinostat
Mocetinostat is studied according to the same protocol as in Example 37. Patients are randomly allocated to Mocetinostat or placebo treatment for a median duration of 18 months. In- hospital treatment is initiated as an intravenous infusion of Mocetinostat followed by oral treatment with 2 mg Mocetinostat daily.
The treatment shows that Mocetinostat can effectively reduce the risk for future major cardiovascular events. The risk is reduced by approximately 30% in addition to that of conventional therapy, i.e. lowers the annual absolute event rate to approximately 5%. Thus, this study confirms the clinical efficacy and feasibility of using Mocetinostat for secondary prevention of cardiovascular events.
REFERENCE EXAMPLE 44
Clinical outcome study in unstable angina/non-ST segment elevation myocardial infarction using SB939
SB939 is studied according to the same protocol as in Example 37. Patients are randomly allocated to SB939 or placebo treatment for a median duration of 18 months. In-hospital treatment is initiated as an intravenous infusion of SB939 followed by oral treatment with 0.4 mg SB939 daily.
The treatment shows that SB939 can effectively reduce the risk for future major cardiovascular events. The risk is reduced by approximately 30% in addition to that of conventional therapy, i.e. lowers the annual absolute event rate to approximately 5%. Thus, this study confirms the clinical efficacy and feasibility of using SB939 for secondary prevention of cardiovascular events.
REFERENCE EXAMPLE 45
Clinical outcome study in unstable angina/non-ST segment elevation myocardial infarction using CXD101
CXD101 is studied according to the same protocol as in Example 37. Patients are randomly allocated to 10 mg CXD101 daily or placebo treatment for a median duration of 18 months. In-hospital treatment is initiated as an intravenous infusion of CXD101 followed by oral treatment with 10 mg CXD101 daily.
The treatment shows that CXD101 can effectively reduce the risk for future major cardiovascular events. The risk is reduced by approximately 30% in addition to that of conventional therapy, i.e. lowers the annual absolute event rate to approximately 5%. Thus, this study confirms the clinical efficacy and feasibility of using CXD101 for secondary prevention of cardiovascular events.
REFERENCE EXAMPLE 46
Primary preventive clinical outcome study using Vorinostat
The third outcome study investigates the primary preventive effect of Vorinostat in healthy subjects with an increased risk for atherothrombotic cardiovascular events i.e. cigarette smoking, abnormal blood lipid levels, hypertension, diabetes, abdominal obesity, low-grade inflammation and/or atherosclerosis. Subjects are randomized to double-blind oral treatment with 10 mg Vorinostat or placebo daily. The risk of a primary atherothrombotic event is followed annually. The primary composite efficacy endpoint is mortality, or non-fatal myocardial infarction or ischemic stroke. The study is event-driven to a total of 180 events in the placebo group.
In this population the annual event rate is reduced by 30% from 1.5 to 1%. The treatment effect shows that Vorinostat can reduce the risk for future cardiovascular events in healthy high-risk subjects and that Vorinostat is suitable for primary prevention of cardiovascular events.
REFERENCE EXAMPLE 47
Primary preventive clinical outcome study using Belinostat
Belinostat is studied according to the same protocol as in Example 46. Patients are randomized to 65 mg Belinostat or placebo daily.
In this population the annual event rate is reduced by 30% from 1.5 to 1%. The treatment effect shows that Belinostat can reduce the risk for future cardiovascular events in healthy high-risk subjects and that Belinostat is suitable for primary prevention of cardiovascular events.
REFERENCE EXAMPLE 48
Primary preventive clinical outcome study using Givinostat
Givinostat is studied according to the same protocol as in Example 46. Patients are randomized to 2 mg Givinostat or placebo daily.
In this population the annual event rate is reduced by 30% from 1.5 to 1%. The treatment effect shows that Givinostat can reduce the risk for future cardiovascular events in healthy high-risk subjects and that Givinostat is suitable for primary prevention of cardiovascular events.
REFERENCE EXAMPLE 49
Primary preventive clinical outcome study using Panobinostat
Panobinostat is studied according to the same protocol as in Example 46.
Patients are randomized to 0.5 mg Panobinostat or placebo daily.
In this population the annual event rate is reduced by 30% from 1.5 to 1%. The treatment effect shows that Panobinostat can reduce the risk for future cardiovascular events in healthy high-risk subjects and that Panobinostat is suitable for primary prevention of cardiovascular events.
REFERENCE EXAMPLE 50
Primary preventive clinical outcome study using PCI-24781
PCI-24781 is studied according to the same protocol as in Example 46. Patients are randomized 2 mg PCI-24781 or placebo daily.
In this population the annual event rate is reduced by 30% from 1.5 to 1%. The treatment effect shows that PCI-24781 can reduce the risk for future cardiovascular events in healthy high-risk subjects and that PCI-24781 is suitable for primary prevention of cardiovascular events.
REFERENCE EXAMPLE 51
Primary preventive clinical outcome study using JNJ-26481585
JNJ-26481585 is studied according to the same protocol as in Example 46. Patients are randomized 0.2 mg JNJ-26481585 or placebo daily.
In this population the annual event rate is reduced by 30% from 1.5 to 1%. The treatment effect shows that JNJ-26481585 can reduce the risk for future cardiovascular events in healthy high-risk subjects and that JNJ-26481585 is suitable for primary prevention of cardiovascular events.
REFERENCE EXAMPLE 52
Primary preventive clinical outcome study using Mocetinostat
Mocetinostat is studied according to the protocol in Example 46. Patients are randomized to 2mg Mocetinostat or placebo daily.
In this population the annual event rate is reduced by 30% from 1.5 to 1%. The treatment effect shows that Mocetinostat can reduce the risk for future cardiovascular events in healthy high-risk subjects and that Mocetinostat is suitable for primary prevention of cardiovascular events.
REFERENCE EXAMPLE 53
Primary preventive clinical outcome study using SB939
SB939 is studied according to the same protocol as in Example 46. Patients are randomized to 0.4 mg SB939 or placebo daily.
In this population the annual event rate is reduced by 30% from 1.5 to 1%. The treatment effect shows that SB939 can reduce the risk for future cardiovascular events in healthy high-risk subjects and that SB939 is suitable for primary prevention of cardiovascular events.
REFERENCE EXAMPLE 54
Primary preventive clinical outcome study using CXD101
CXD101 is studied according to the same protocol as in Example 46. Patients are randomized to 10 mg CXD101 or placebo daily.
In this population the annual event rate is reduced by 30% from 1.5 to 1%. The treatment effect shows that CXD101 can reduce the risk for future cardiovascular events in healthy high-risk subjects and that CXD101 is suitable for primary prevention of cardiovascular events.
REFERENCE EXAMPLE 55
Clinical outcome study of Vorinostat in high-risk patients for prevention of recurrent venous thromboembolic events
This study is performed in high-risk patients who have experienced a recent deep vein thrombosis or circulatory stable pulmonary embolus to investigate the preventive effect of Vorinostat treatment on the risk for recurrent venous thrombotic events. Patients with a cancer diagnosis who presents with a first episode of a proximal deep venous thrombosis without unstable pulmonary embolism will be included. The patients will receive conventional treatment (i.e warfarin for 3-6 months) and thereafter included in the study. Patients are randomized in a parallel study design to receive double-blind oral treatment with 10 mg Vorinostat or placebo daily, in addition to optimal conventional treatment. The event rate is monitored by Kaplan-Meyer statistics. The primary efficacy endpoint is the composite measure of either mortality, or recurrent deep venous thrombosis or pulmonary embolism. The study is event-driven to a total of 180 events in the placebo group. The study shows that long-term Vorinostat treatment according to the invention herein reduces this risk by approximately 30% in addition to that of conventional therapy. Thus, this study confirms the clinical efficacy and feasibility of using Vorinostat for secondary prevention of venous thromboembolism.
REFERENCE EXAMPLE 56
Clinical outcome study of Belinostat in high-risk patients for prevention of recurrent venous thromboembolic events
Belinostat is studied according to the same protocol as in example 55. Patients are randomized in a parallel study design to receive double-blind oral treatment with 10 mg Vorinostat or placebo daily.
The study shows that long-term Belinostat treatment according to the invention herein reduces this risk by approximately 30% in addition to that of conventional therapy. Thus, this study confirms the clinical efficacy and feasibility of using Belinostat for secondary prevention of venous thromboembolism.
REFERENCE EXAMPLE 57
Clinical outcome study of Givinostat in high-risk patients for prevention of recurrent venous thromboembolic events
Givinostat is studied according to the same protocol as in example 55. Patients are randomized in a parallel study design to receive double-blind oral treatment with 2 mg Givinostat or placebo daily.
The study shows that long-term Givinostat treatment according to the invention herein reduces this risk by approximately 30% in addition to that of conventional therapy. Thus, this study confirms the clinical efficacy and feasibility of using Givinostat for secondary prevention of venous thromboembolism.
REFERENCE EXAMPLE 58
Clinical outcome study of Panobinostat in high-risk patients for prevention of recurrent venous thromboembolic events
Panobinostat is studied according to the same protocol as in example 55. Patients are randomized in a parallel study design to receive double-blind oral treatment with 0.5 mg Panobinostat or placebo daily.
The study shows that long-term Panobinostat treatment according to the invention herein reduces this risk by approximately 30% in addition to that of conventional therapy. Thus, this study confirms the clinical efficacy and feasibility of using Panobinostat for secondary prevention of venous thromboembolism.
REFERENCE EXAMPLE 59
Clinical outcome study of PCI-24781 in high-risk patients for prevention of recurrent venous thromboembolic events
PCI-24781 is studied according to the same protocol as in example 55. Patients are randomized in a parallel study design to receive double-blind oral treatment with 2 mg PCI-24781 or placebo daily.
The study shows that long-term PCI-24781 treatment according to the invention herein reduces this risk by approximately 30% in addition to that of conventional therapy. Thus, this study confirms the clinical efficacy and feasibility of using PCI-24781 for secondary prevention of venous thromboembolism.
REFERENCE EXAMPLE 60
Clinical outcome study of JNJ-26481585 in high-risk patients for prevention of recurrent venous thromboembolic events
JNJ-26481585 is studied according to the same protocol as in example 55. Patients are randomized in a parallel study design to receive double-blind oral treatment with 0.2 mg JNJ-26481585 or placebo daily.
The study shows that long-term JNJ-26481585 treatment according to the invention herein reduces this risk by approximately 30% in addition to that of conventional therapy. Thus, this study confirms the clinical efficacy and feasibility of using JNJ-26481585 for secondary prevention of venous thromboembolism.
REFERENCE EXAMPLE 61
Clinical outcome study of Mocetinostat in high-risk patients for prevention of recurrent venous thromboembolic events
Mocetinostat is studied according to the same protocol as in example 55. Patients are randomized in a parallel study design to receive double-blind oral treatment with 2 mg Mocetinostat or placebo daily.
The study shows that long-term Mocetinostat treatment according to the invention herein reduces this risk by approximately 30% in addition to that of conventional therapy. Thus, this study confirms the clinical efficacy and feasibility of using Mocetinostat for secondary prevention of venous thromboembolism.
REFERENCE EXAMPLE 62
Clinical outcome study of SB939 in high-risk patients for prevention of recurrent venous thromboembolic events
SB939 is studied according to the same protocol as in example 55. Patients are randomized in a parallel study design to receive double-blind oral treatment with 0.4 mg SB939 or placebo daily.
The study shows that long-term SB939 treatment according to the invention herein reduces this risk by approximately 30% in addition to that of conventional therapy. Thus, this study confirms the clinical efficacy and feasibility of using SB939 for secondary prevention of venous thromboembolism.
REFERENCE EXAMPLE 63
Clinical outcome study of CXD101 in high-risk patients for prevention of recurrent venous thromboembolic events
CXD101 is studied according to the same protocol as in example 55. Patients are randomized in a parallel study design to receive double-blind oral treatment with 10 mg CXD101 or placebo daily.
The study shows that long-term CXD101 treatment according to the invention herein reduces this risk by approximately 30% in addition to that of conventional therapy. Thus, this study confirms the clinical efficacy and feasibility of using CXD101 for secondary prevention of venous thromboembolism.
EXAMPLE 64
To determine if substance X is an interesting HDACi, screening for activity towards a panel of recombinant human HDAC enzymes HDAC1-11) is performed in collaboration with Reaction Biology Corporation. In these studies a dilution series of compound X is generated with ten steps of three-fold dilutions starting at 10 µM (e.g 10 µM, 3 µM, 1 µM, 300 nM, 100 nM, 30 nM, 10 nM, 3 nM, 1 nM, 0.3 nM) and this is plotted in a dose-response curve to yield the IC₅₀ value.
EXAMPLE 65
In a next step (following the procedure of Example 64), interesting substances can be tested for HDAC-inhibitory activity in cultured human umbilical vein endothelial cells (HUVEC) at three doses: 10 x IC₅₀, 1 x IC₅₀ and 0.1 x IC₅₀ . If no IC50 value has been obtained, the dilution series in the previous example can be used instead of the 10 x, 1x and 0.1x IC50 for the analysis. Readouts are cytotoxicity (LDH assay Promega), HDAC activity (HDAC activity assay kit from Active Motif), increased histone acetylation (as measured by western blot with pan-acetylated histone H3/H4 antibodies), and effect on t-PA mRNA levels (real-time PCR).
REFERENCE EXAMPLE 66
Dose escalation study for Vorinostat. A dose escalation study for Vorinostat is performed starting oral treatment at 10 mg/day in the first cohort (5 subjects per cohort) and then increased in 100% increments (10, 20, 40,... mg/day) until the desired plasma concentration of 100 nM is observed. Venous blood samples are collected at time points 0, 0.25, 0.5, 1, 2, 4, 6, 8, 10, 12, 16, 24, and 48 h after dosing. The concentration of Vorinostat in the blood samples are determined using liquid chromatography-tandem mass spectrometry (LC-MS) (Kelly WK. et al. (2005) Phase I study of an oral histone deacetylase inhibitor, suberoylanilide hydroxamic acid, in patients with advanced cancer. J Clin Oncol 23: 3923-3931.)
REFERENCE EXAMPLE 67
Dose escalation study Belinostat. A dose escalation study for Belinostat is performed starting oral treatment at 50 mg/day in the first cohort (5 subjects per cohort) and then increased in 100% increments (50, 100, 200, 400... mg/day) until the desired plasma concentration of 200 nM is observed. Venous blood samples are collected at time points 0, 0.25, 0.5, 1, 2, 4, 6, 8, 10, 12, 16, 24, and 48 h after dosing. The concentration of Belinostat in the blood samples are determined using liquid chromatography-tandem mass spectrometry (LC-MS/MS).(Steele NL, Plumb JA, Vidal L, Tjornelund J, Knoblauch P, et al. (2008) A phase 1 pharmacokinetic and pharmacodynamic study of the histone deacetylase inhibitor belinostat in patients with advanced solid tumors. Clin Cancer Res 14: 804-810.).
REFERENCE EXAMPLE 68
Dose escalation study Givinostat. A dose escalation study for Givinostat is performed starting oral treatment at 5 mg/day in the first cohort (5 subjects per cohort) and then increased in 100% increments (5, 10, 20, 40... mg/day) until the desired plasma concentration of 50 nM is observed. Venous blood samples are collected at time points 0, 0.25, 0.5, 1, 2, 4, 6, 8, 10, 12, 16, 24, and 48 h after dosing. The concentration of Givinostat in the blood samples are determined using liquid chromatography-tandem mass spectrometry (LC-MS/MS) (Furlan A, et al. (2011) Pharmacokinetics, Safety and Inducible Cytokine Responses during a Phase 1 Trial of the Oral Histone Deacetylase Inhibitor ITF2357 (Givinostat). Mol Med 17: 353-362.)
A dose escalation study for Givinostat is performed starting at 1 mg/day in the first cohort (5 subjects per cohort) and then increased in 100% increments (1, 2, 4, 8, 16... mg/day) until the desired plasma concentration of 25 nM is observed. Venous blood samples are collected at time points 0, 0.25, 0.5, 1, 2, 4, 6, 8, 10, 12, 16, 24, and 48 h after dosing. The concentration of Givinostat in the blood samples are determined using liquid chromatography-tandem mass spectrometry (LC-MS/MS).
REFERENCE EXAMPLE 69
Dose escalation study Panobinostat. A dose escalation study for Panobinostat is performed starting oral treatment at 0.5 mg/day in the first cohort (5 subjects per cohort) and then increased in 100% increments (0.5, 1, 2, 4, 8... mg/day) until the desired plasma concentration of 5 nM is observed. Venous blood samples are collected at time points 0, 0.25, 0.5, 1, 2, 4, 6, 8, 10, 12, 16, 24, and 48 h after dosing. The concentration of Panobinostat in the blood samples are determined using liquid chromatography-tandem mass spectrometry (LC-MS/MS).
EXAMPLE 70
Effect of VPA on inflammation-induced t-PA suppression *in vitro*
We have previously shown that proinflammatory cytokines e.g. TNF-alpha and IL-1b suppress t-PA production in endothelial cells. We then investigated if VPA could reverse a TNF-alpha suppressed t-PA response. Human umbilical vein endothelial cells (HUVECs) were prepared by collagenase treatment of fresh umbilical cords (Jaffe, E.A., et al. J Clin Invest 52, 2745-2756 (1973)) obtained from the maternity ward of the Sahlgrenska University hospital, Gothenburg, Sweden. Cells were cultured in EGM-2 medium (Lonza, Basel, Switzerland) and all experiments were performed in passage 1 of subcultivation. Confluent HUVECs were pre-treated with 0.1 ng/ml human recombinant TNF-alpha (Sigma-Aldrich) for 24 hours then exposed to optimal concentrations VPA in complete medium. After incubation with the VPA and TNF-alpha for an additional 24 hours, cells and conditioned media were harvested.
Total RNA was prepared using RNeasy Mini RNA kit (Qiagen, Hilden, Germany) and genomic DNA was removed using RNase-free DNase I set (Qiagen). Levels of t-PA mRNA were analyzed with real-time RT-PCR, performed on an Applied Biosystems 7500 Fast Real-Time PCR System using cDNA and Taqman reagents obtained from Applied Biosystems (Foster City, CA, USA). Hypoxanthine phosphoribosyl transferase (HPRT, Assay number Hs99999909_m1, Applied Biosystems) was used as endogenous internal standard.
Endothelial cells in culture are known to constitutively secrete the majority of synthesized t-PA making conditioned media a suitable source for quantification of t-PA protein. Conditioned medium from cell cultures was collected, centrifuged (10,000 x g, 10 min, 4 °C) to remove cell debris, transferred to fresh tubes and stored at -70 °C. Concentrations of t-PA antigen in conditioned media were determined using the commercially available TriniLize t-PA antigen ELISA (Trinity Biotech, Bray, Ireland) according to the manufacturer's protocol.
0.1 ng/ml of TNF-alpha suppressed t-PA mRNA production 2-fold. Low concentrations of VPA reversed this suppression and complete normalization was achieved with 0.35 mM of the substance (Fig 1). Corresponding results are also seen at the level of secreted t-PA protein.
EXAMPLE 71
Shift of the VPA dose-response curve in the presence of TNF-alpha
In an attempt to mimic the potentially highly inflamed conditions in the local microenvironment surrounding an atherosclerotic plaque, endothelial cells were exposed to a high concentration (10 ng/ml) of TNF-alpha for 24 hours and then VPA was added for an additional 24 h. Cells were treated and mRNA prepared as described in example 1.
When comparing VPA dose-response curves for control and TNF-alpha treated cells we surprisingly observed a difference in the response-pattern to VPA in the absence and presence of TNF. In control cells the maximum efficacy of VPA in inducing t-PA was about 10-fold. In TNF-alpha treated cells, on the other hand, the maximum efficacy was strongly enhanced to about 50-fold (Fig. 2), demonstrating that lower doses than expected of VPA can improve or normalize an inflammation-suppressed fibrinolytic function.
EXAMPLE 72
Intermediate endpoint study: Effects of VPA on *in vivo* t-PA release in man
An intermediate endpoint proof-of-concept study is performed in patients with atherosclerotic disease and signs of a low-grade inflammatory condition investigated before and after treatment with valproic acid.
The study comprises 16 patients with stable angina pectoris and elevated serum levels of high-sensitivity C-reactive protein (hs-CRP) > 3mg/L. Patients are investigated before and after oral treatment with 100 mg valproic acid twice daily for 2 weeks. The study has a randomized, cross-over design and t-PA release capacity is investigated before and after treatment, with each individual serving as his/her own control.
The capacity for t-PA release is investigated in the perfused-forearm model that we have developed, which is the only method that permit a direct measurement of the local release of t-PA from the endothelium (Hrafnkelsdottir, T., et al. Lancet 352, 1597-1598 (1998), Wall, U., et al. Blood 91, 529-537 (1998). Since t-PA has a rapid hepatic clearance, it is impossible to infer endothelial release rates from plasma levels obtained from standard venous samples. With the invasive model, however, net forearm t-PA release rates are calculated from arterio-venous concentration gradients of t-PA after correction for forearm plasma flow. Acute t-PA release responses are induced by intraarterial infusions of Substance P (Bachem, Bubendorf, Switzerland), and the amount and protein secretion profile is used as a measure of t-PA release capacity.
Comparison of the t-PA secretion profiles before and after treatment with VPA shows that the total amount of t-PA released is increased by approximately 50%. This study shows that there is a significant improvement of the cumulative amount of t-PA released across the forearm vasculature in response to the stimulation after short-term treatment with a low dose of VPA in patients with low-grade systemic inflammation.
EXAMPLE 73
Clinical outcome study using VPA in high-risk patients for prevention of recurrent events The first clinical outcome study is performed in high-risk patients who have experienced a recent major atherothrombotic cardiovascular event (myocardial infarction or ischemic stroke) to investigate the preventive effect of VPA treatment on the risk for recurrent events. Signs of a low-grade inflammatory condition is an inclusion criterion, defined as an elevated serum level of high-sensitivity C-reactive protein (hs-CRP) > 3mg/L. The annual risk for a recurrent atherothrombotic event in the investigated population is estimated to approximately 7%. Patients are randomized in a parallel study design to receive double-blind oral treatment with 100 mg valproic acid or placebo twice daily, in addition to optimal conventional treatment. The event rate is monitored by Kaplan-Meyer statistics. The primary efficacy endpoint is the composite measure of either mortality, or non-fatal myocardial infarction or ischemic stroke. The study is event-driven to a total of 180 events in the placebo group. The study shows that long-term VPA treatment according to the invention herein reduces this risk by approximately 30% in addition to that of conventional therapy, i.e. lowers the annual absolute event rate to approximately 5%. Thus, this study confirms the clinical efficacy and feasibility of using VPA for secondary prevention of cardiovascular events.
EXAMPLE 74
Clinical outcome study in unstable angina/non-ST segment elevation myocardial infarction using VPA
The second clinical outcome study is performed in patients with non-ST-segment elevation acute coronary syndromes. This study is a randomized, double-blind trial enrolling 7000 patients within 72 hours of presentation with either unstable angina or non-ST segment elevation myocardial infarction who are not intended to undergo revascularization procedures for their index event. Patients are randomly allocated to valproic acid or placebo treatment for a median duration of 18 months. In-hospital treatment is initiated as an intravenous infusion of valproic acid followed by oral treatment with 100 mg valproic acid twice daily. The primary composite efficacy endpoint is the time to first occurrence of cardiovascular death, myocardial infarction, or stroke. The study shows that VPA treatment reduces this risk by approximately 30% in addition to that of conventional therapy. Thus, this study confirms the clinical efficacy and feasibility of using VPA for secondary prevention of cardiovascular events in patients with unstable coronary artery disease.
EXAMPLE 75
Primary preventive clinical outcome study using VPA
The third outcome study investigates the primary preventive effect of VPA in healthy subjects with an increased risk for atherothrombotic cardiovascular events due to low-grade inflammation. The inflammatory activation is clinically defined as an elevated serum level of high-sensitivity C-reactive protein (hs-CRP) > 3mg/L. Subjects are randomized to double-blind oral treatment with 100 mg valproic acid or placebo twice daily. The risk of a primary atherothrombotic event is followed annually. The primary composite efficacy endpoint is mortality, or non-fatal myocardial infarction or ischemic stroke. The study is event-driven to a total of 180 events in the placebo group. In this population the annual event rate is reduced by 30% from 1.5 to 1%. The treatment effect shows that VPA can reduce the risk for future cardiovascular events in healthy high-risk subjects and that VPA is suitable for primary prevention of cardiovascular events.
EXAMPLE 76
Clinical outcome study using VPA in high-risk patients for prevention of recurrent venous thromboembolic events
This study is performed in high-risk patients who have experienced a recent deep vein thrombosis or circulatory stable pulmonary embolus to investigate the preventive effect of VPA treatment on the risk for recurrent venous thrombotic events. Patients with a cancer diagnosis and low grade inflammation who present with a first episode of a proximal deep venous thrombosis without unstable pulmonary embolism are included. The patients receive conventional treatment (i.e warfarin for 3-6 months) and thereafter are included in the study. Patients are randomized in a parallel study design to receive double-blind oral treatment with 100 mg valproic acid or placebo twice daily, in addition to optimal conventional treatment. The event rate is monitored by Kaplan-Meyer statistics. The primary efficacy endpoint is the composite measure of either mortality, or recurrent deep venous thrombosis or pulmonary embolism. The study is event-driven to a total of 180 events in the placebo group. The study shows that long-term VPA treatment according to the invention herein reduces this risk by approximately 30% in addition to that of conventional therapy. Thus, this study confirms the clinical efficacy and feasibility of using VPA for secondary prevention of venous thromboembolism.
REFERENCE EXAMPLE 77
HUVECs are treated with different concentrations of first generation hydroxamates (TSA), second generation hydroxamates (Givinostat, Vorinostat, Belinostat, Panobinostat, SB939, PCI24781), benzamides (Mocetinostat, Entinostat) or short chain fatty acids (SCFA, Butyrate, Phenylbutyrate) for 24 h and t-PA mRNA was measured. The doses giving a 100% increase of t-PA mRNA (C100) was determined and compared to the maximum plasma concentration (Cmax) achieved when the maximum tolerated dose (MTD) of each substance is administered to humans, by dividing the C100 with the Cmax. For the first generation hydroxamate TSA this comparison is impossible as it is unsuitable for use in humans, hence, no such comparison is made. For the second generation hydroxamates we find that this ratio is significantly lower than for the benzamide and short chain fatty acid class surprisingly indicating that the second generation hydroxamates stimulate t-PA expression at relatively lower concentrations compared to the other classes tested (see Table A below). In the table below the values for MTD and Cmax are from the following references (mentioned in the same order as in the table): Steele, N.L. et al Cancer Chemother Pharmacol 67(6):1273-9 (2011), Kelly, K.K. et al J Clin Oncol 23:3923-3931 (2005), Furlan, A. et al Mol Med 17(5-6) 353-362 (2011), Fukutomi, A. et al Invest New Drugs 2011 April 12, Yong, W.P. et al Ann Oncol 22(11) 2516-22 (2011), Garcia-Manero, G. et al. Blood 112: 981-989 (2008), Ryan, Q.C. et al J Clin Oncol 23(17): 3912-3922 (2005), Edelman, M.J. et al Cancer Chemother Pharmacol 51: 439-444, http://www.drugs.com/pro/buphenyl.html *Butyrate was administered in the prodrug form tributyrin.

**Table A**

| **HDACi** | **Class** | **MTD** | **Cmax (ng/ml)** | **Cmax (µM)** | **C100 tPA (µM)** | **C100/Cmax** |
|---|---|---|---|---|---|---|
| Belinostat | 2:nd Hydrox | 1000 mg/m2 | ∼1400 | 4 µM | 0.2 | 0.05 |
| Vorinostat | 2:nd Hydrox | 200 mg b.i.d | 300 | 1,1 µM | 0.1 | 0.09 |
| Givinostat | 2:nd Hydrox | 200 mg | 300 | 0,65 µM | 0.05 | 0.08 |
| Panobinostat | 2:nd Hydrox | 20 mg | 20 | 0,06 µM | 0.004 | 0.07 |
| SB-939 | 2:nd Hydrox | 80 mg | ∼400 | 1.1 µM | 0.05 | 0.05 |
| Mocetinostat | Benzamide | 60 mg/m2 | 200 | 0,5 µM | 0.1 | 0.2 |
| Entinostat | Benzamide | 10 mg/m2 | 45 | 0,12 µM | 0.3 | 2.5 |
| Butyrate* | SCFA | 200 mg/kg t.i.d | | 0.1 mM | 0.1 mM | 1.0 |
| Phenylbutyrate | SCFA | 5 g | 218 000 | 1.1 mM | 1.3 mM | 1.2 |

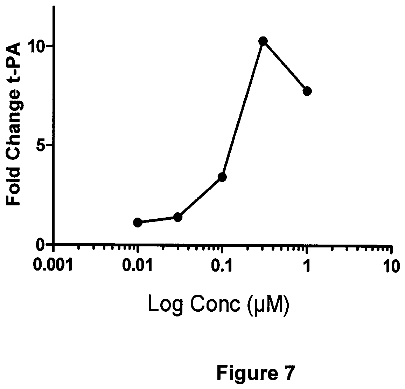

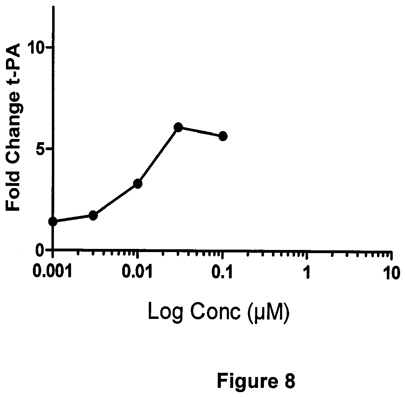

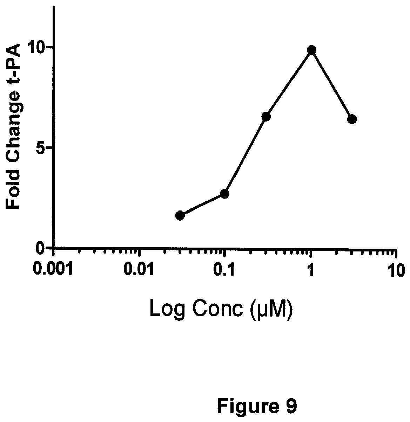

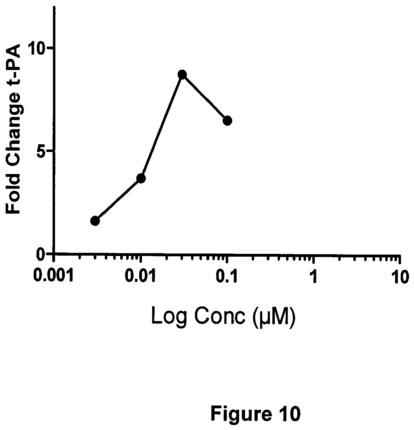

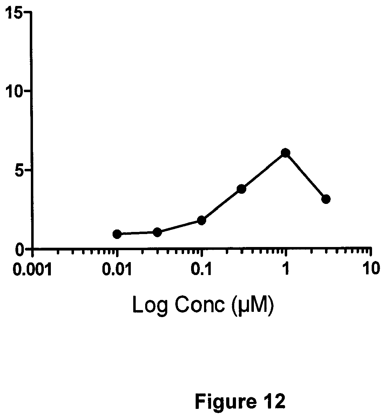

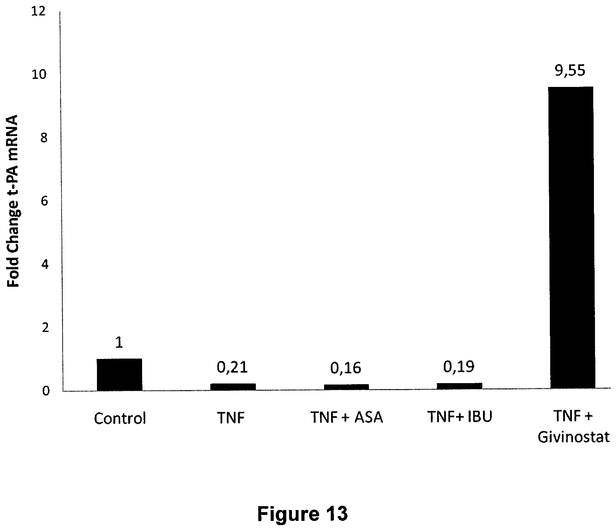

REFERENCE EXAMPLE 78
HUVECs were treated with TNF-alpha (TNF-a) for 1 h and then optimal concentrations of the anti.inflammatory substances acetylsalicylic acid (ASA, 1 mM) and ibuprofen (IBU, 1 mM) was added. Givinostat was also added to the cells for comparison. Cells were harvested and t-PA mRNA levels analysed according to example 1. TNF-a suppressed the expression of t-PA five-fold and this was not counteracted by either ASA or IBU. On the other hand, Givinostat was able to not only completely reverse the TNF-mediated suppression but indeed caused a 9-fold increase of t-PA (figure 13). This demonstrates that the effect on t-PA of the HDACi described in the present application is not a result of a general anti-inflammatory effect but suggests an effect mediated by a non-inflammatory pathway.
REFERENCE EXAMPLE 79
*In vitro* dose response experiment for Givinostat
Givinostat was studied according to the protocol described in Example 1. Cells were treated with 10 nM-10 µM of Givinostat for 24 h.
A significant increase of t-PA mRNA levels was seen already at 30 nM of Givinostat (Selleck Chemicals, Houston, TX, USA). The effect on t-PA expression increased in a dose-dependent manner and reached maximum at 0.3 µM where t-PA expression was increased 10 times (figure 7).
REFERENCE EXAMPLE 80
*In vitro* dose response experiment for Panobinostat
Panobinostat was studied according to the protocol described in Example 1. Cells were treated with 1 nM-10 µM of Panobinostat (Selleck Chemicals, Houston, TX, USA) for 24 h.
A significant increase of t-PA mRNA levels was seen already at 3 nM of Panobinostat. The effect on t-PA expression increased in a dose-dependent manner and reached maximum at around 30 nM where t-PA expression increased approximately 10 times (figure 10).
REFERENCE EXAMPLE 81
*In vitro* dose response experiment for PCI-24781
PCI-24781 was studied according to the protocol described in Example 1. Cells were treated with 3 nM-3 µM of PCI-24781 (Selleck Chemicals, Houston, TX, USA) for 24 h. A significant increase of t-PA mRNA levels was seen already at 100 nM of PCI-24781. The effect on t-PA expression was increased in a dose-dependent manner and reached maximum at around 1 µM where t-PA expression increased approximately 6 times (fig 12).
REFERENCE EXAMPLE 82
*In vitro* dose response experiment for JNJ-26481585
JNJ-26481585 was studied according to the protocol described in Example 1. Cells were treated with 1 nM-1 µM of JNJ-26481585 (Selleck Chemicals, Houston, TX, USA) for 24 h.
A significant increase of t-PA mRNA levels was seen already at 3 nM of JNJ-26481585. The effect on t-PA expression increased in a dose-dependent manner and reached a maximum at around 30 nM where t-PA expression was increased approximately 6 times (figure 8).
REFERENCE EXAMPLE 83
*In vitro* dose response experiment for Mocetinostat
Mocetinostat was studied according to the protocol described in Example 1. Cells were treated with 10 nM-10 µM of Mocetinostat (Selleck Chemicals, Houston, TX, USA) for 24 h.
A significant increase of t-PA mRNA levels was seen already at 0.1 µM of Mocetinostat. The effect on t-PA expression is increased in a dose-dependent manner and reached a maximum at around 3 µM where t-PA expression was increased approximately 15 times.
REFERENCE EXAMPLE 84
*In vitro* dose response experiment for SB939
SB939 was studied according to the protocol described in Example 1. Cells were treated with 10 nM-10 µM of SB939 (Selleck Chemicals, Houston, TX, USA) for 24 h.
A significant increase of t-PA mRNA levels was seen already at 30 nM of SB939. The effect on t-PA expression was increased in a dose-dependent manner and reached a maximum at around 1 µM where t-PA expression was increased approximately 10 times (figure 9).

## Claims

1. Valproic acid, or a pharmaceutically acceptable salt thereof, for use in:
(I) improving or normalizing endogenous fibrinolysis impaired by local or systemic inflammation; or
(II) treating or preventing a pathological condition associated with excess fibrin deposition and/or thrombus formation, wherein the pathological condition is caused wholly or at least in part by an increased fibrin deposition and/or reduced fibrinolytic capacity due to local or systemic inflammation,
wherein the valproic acid, or a pharmaceutically acceptable salt thereof, is administered in an amount between 1 µg to 30 mg per kilogram of body weight per day and in the range of approximately 50-1000 mg per day.

2. The compound for use as claimed in Claim 1, wherein the valproic acid, or a pharmaceutically acceptable salt thereof, is administered in an amount that results in a plasma concentration in the range of approximately 0.05-0.3 mM.

3. The compound for use as claimed in Claim 1 or Claim 2, wherein:
(i) the improving or normalizing endogenous fibrinolysis impaired by local or systemic inflammation is part of the treatment or prevention of a cardiovascular disease;
(ii) the pathological condition is a cardiovascular disease.

4. The compound for use as claimed in Claim 3, wherein the pathological condition is selected from the group consisting of myocardial infarction, stable angina pectoris, unstable angina pectoris, intermittent claudication, ischemic stroke, transient ischemic attack, deep vein thrombosis, and pulmonary embolism.

5. The compound for use as claimed in Claim 4, wherein the pathological condition is selected from the group consisting of deep vein thrombosis and pulmonary embolism.

6. The compound for use as claimed in any one of Claims 1 to 5, wherein the amount of valproic acid, or a pharmaceutically acceptable salt thereof, administered yields a Cmax in the range of approximately 0.01-0.7mM.

## Patentansprüche

1. Valproinsäure oder ein pharmazeutisch unbedenkliches Salz davon zur Verwendung in Folgendem:
(I) Verbessern oder Normalisieren endogener Fibrinolyse, die durch lokale oder systemische Entzündungen beeinträchtigt wird; oder
(II) Behandeln oder Vorbeugen eines pathologischen Zustands, der in Zusammenhang mit einer übermäßigen Fibrinablagerung und/oder Thrombusbildung steht, wobei der pathologische Zustand ganz oder wenigstens zum Teil durch eine erhöhte Fibrinablagerung und/oder eine verringerte fibrinolytische Kapazität aufgrund einer lokalen oder systemischen Entzündung verursacht wird,
wobei die Valproinsäure oder ein pharmazeutisch unbedenkliches Salz davon in einer Menge zwischen 1 µg bis 30 mg pro Kilogramm Körpergewicht pro Tag und in dem Bereich von ungefähr 50-1000 mg pro Tag verabreicht wird.

2. Verbindung zur Verwendung nach Anspruch 1, wobei die Valproinsäure oder ein pharmazeutisch unbedenkliches Salz davon in einer Menge verabreicht wird, die zu einer Plasmakonzentration in dem Bereich von ungefähr 0,05-0,3 mM führt.

3. Verbindung zur Verwendung nach Anspruch 1 oder 2, wobei:
(i) das Verbessern oder Normalisieren endogener Fibrinolyse, die durch lokale oder systemische Entzündungen beeinträchtigt wird, Teil der Behandlung oder Vorbeugung einer Herz-Kreislauf-Erkrankung ist;
(ii) der pathologische Zustand eine Herz-Kreislauf-Erkrankung ist.

4. Verbindung zur Verwendung nach Anspruch 3, wobei der pathologische Zustand aus der Gruppe ausgewählt ist, die aus Myokardinfarkt, stabiler Angina pectoris, instabiler Angina pectoris, Claudicatio intermittens, ischämischem Schlaganfall, vorübergehendem ischämischem Anfall, tiefer Venenthrombose und Lungenembolie besteht.

5. Verbindung zur Verwendung nach Anspruch 4, wobei der pathologische Zustand aus der Gruppe ausgewählt ist, die aus tiefer Venenthrombose und Lungenembolie besteht.

6. Verbindung zur Verwendung nach einem der Ansprüche 1 bis 5, wobei die verabreichte Menge von Valproinsäure oder einem pharmazeutisch unbedenklichen Salz davon eine Cmax in dem Bereich von ungefähr 0,01-0,7 mM ergibt.

## Revendications

1. Acide valproïque, ou un sel pharmaceutiquement acceptable de celui-ci, à utiliser dans :
(I) l'amélioration ou la normalisation de la fibrinolyse endogène altérée par une inflammation locale ou systémique ; ou
(II) le traitement ou la prévention d'un état pathologique associé à un dépôt excessif de fibrine et/ou à la formation de thrombus, dans lequel l'état pathologique est causé en tout ou au moins en partie par un dépôt accru de fibrine et/ou une capacité fibrinolytique réduite due à une inflammation locale ou systémique,
dans lequel l'acide valproïque, ou un sel pharmaceutiquement acceptable de celui-ci, est administré en une quantité comprise entre 1 µg et 30 mg par kilogramme de poids corporel par jour et dans la plage d'environ 50 à 1 000 mg par jour.

2. Composé à utiliser selon la revendication 1, dans lequel l'acide valproïque, ou un sel pharmaceutiquement acceptable de celui-ci, est administré en une quantité qui conduit à une concentration plasmatique dans la plage d'environ 0,05 à 0,3 mM.

3. Composé à utiliser selon la revendication 1 ou la revendication 2, dans lequel :
(i) l'amélioration ou la normalisation de la fibrinolyse endogène altérée par une inflammation locale ou systémique fait partie du traitement ou de la prévention d'une maladie cardiovasculaire ;
(ii) l'état pathologique est une maladie cardiovasculaire.

4. Composé à utiliser selon la revendication 3, dans lequel l'état pathologique est choisi dans le groupe constitué par l'infarctus du myocarde, l'angine de poitrine stable, l'angine de poitrine instable, la claudication intermittente, l'AVC ischémique, l'accident ischémique transitoire, la thrombose veineuse profonde et l'embolie pulmonaire.

5. Composé à utiliser selon la revendication 4, dans lequel l'état pathologique est choisi dans le groupe constitué par la thrombose veineuse profonde et l'embolie pulmonaire.

6. Composé à utiliser selon l'une quelconque des revendications 1 à 5, dans lequel la quantité d'acide valproïque, ou d'un sel pharmaceutiquement acceptable de celui-ci, administrée, donne une Cmax dans la plage d'environ 0,01 à 0,7 mM.
